(19) Europäisches Patentamt
European Patent Office
Office européen des brevets

(11) Numéro de publication: **0 394 101 B1**

(12) # FASCICULE DE BREVET EUROPEEN

(45) Date de publication de fascicule du brevet: **24.08.94**

(51) Int. Cl.[5]: **C07D 281/10**, A61K 31/55

(21) Numéro de dépôt: **90400997.4**

(22) Date de dépôt: **11.04.90**

Le dossier contient des informations techniques présentées postérieurement au dépôt de la demande et ne figurant pas dans le présent fascicule.

(54) **Dérivés de la benzothiazépine, leur procédé de préparation et les intermédiaires ainsi obtenus, leur application comme médicaments et les compositions les renfermant.**

(30) Priorité: **13.04.89 FR 8904905**

(43) Date de publication de la demande:
**24.10.90 Bulletin 90/43**

(45) Mention de la délivrance du brevet:
**24.08.94 Bulletin 94/34**

(84) Etats contractants désignés:
**CH DE FR GB IT LI NL**

(56) Documents cités:
**EP-A- 0 262 373**
**US-A- 3 075 967**
**US-A- 3 330 823**

**EUROPEAN JOURNAL OF MEDICINAL CHEMISTRY - CHIMICA THERAPEUTICA, vol. 9, no. 4, juillet-août 1979, pages 376-380, Paris, FR; J. HUET et al.: "Ouelques dérivés de la tétrahydro-2,3,4,5 benzo [b] 1H-azépine à propriétés antiarythmiques"**

(73) Titulaire: **ROUSSEL-UCLAF**
**35, Boulevard des Invalides**
**F-75007 Paris (FR)**

(72) Inventeur: **Clemence, François**
**2, rue Turgot**
**F-75009 Paris (FR)**
Inventeur: **Frechet, Daniel**
**45, rue Lecourbe**
**F-75015 Paris (FR)**
Inventeur: **Hamon, Gilles**
**7, Boulevard de l'Ouest**
**F-93340 Le Raincy (FR)**
Inventeur: **Jouquey, Simone**
**137, rue des Pyrénées**
**F-75020 Paris (FR)**

(74) Mandataire: **Bourgouin, André et al**
**Département des Brevets**
**ROUSSEL UCLAF**
**111, route de Noisy**
**B.P. no 9**
**F-93230 Romainville (FR)**

**EP 0 394 101 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention a pour objet, les produits de formule générale (I) :

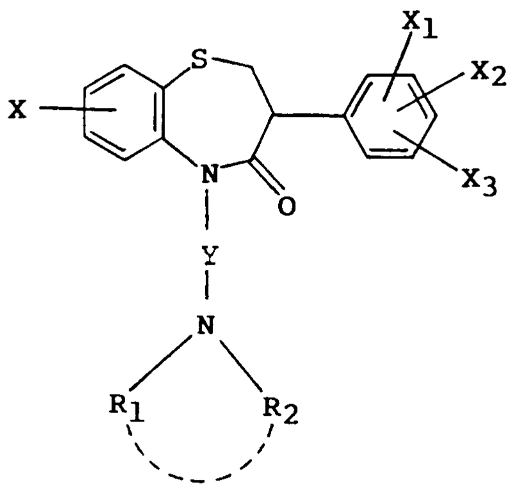

(I)

dans laquelle X représente un atome d'hydrogène, un atome d'halogène, un radical hydroxyle, un radical alkyle ou alkyloxy, renfermant de 1 à 3 atomes de carbone, un radical nitro, amino, monoalkyle ou dialkylamino, ou un radical trifluorométhyle,
$X_1$, $X_2$ et $X_3$ identiques ou différents représentent un atome d'hydrogène, un atome d'halogène, un radical hydroxyle, un radical alkyle ou alkyloxy renfermant de 1 à 3 atomes de carbone, un radical nitro, amino, monoalkyle ou dialkylamino, un radical trifluorométhyle, trifluorométhylthio ou trifluorométhoxy,
Y représente soit le groupement

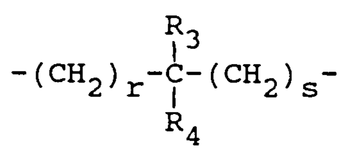

dans lequel
r et s identiques ou différents sont des entiers de 0 à 4, étant entendu que la somme de r et s doit être un entier de 1 à 4,
$R_3$ et $R_4$ identiques ou différents représentent un atome d'hydrogène ou un radical alkyle renfermant de 1 à 4 atomes de de carbone,
soit le groupement

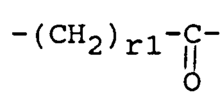

dans lequel r1 représente un entier de 1 à 4,
$R_1$ et $R_2$ identiques ou différents représentent un atome d'hydrogène ou un radical alkyle renfermant de 1 à 4 atomes de carbone éventuellement substitué soit par un radical aryle renfermant de 6 à 12 atomes de carbone lui-même éventuellement substitué par 1, 2 ou 3 radicaux choisis parmi les radicaux alkyle, alkyloxy renfermant de 1 à 4 atomes de carbone, hydroxy, cyano et halogène, soit par une amine éventuellement mono ou disubstituée par un radical alkyle renfermant de 1 à 4 atomes de carbone, ou $R_1$ et $R_2$ forment avec l'atome d'azote auquel ils sont liés un radical hétérocyclique saturé à cinq, six ou sept chaînons comprenant éventuellement un autre hétéroatome choisi parmi les atomes d'azote, d'oxygène ou de soufre et éventuellement substitué, lesdits composés de formule (I) pouvant être sous toutes les formes isomères possibles, racémiques ou optiquement actives ainsi que les sels d'addition avec les acides minéraux ou organiques.

La demande de brevet européen EP 0262373 décrit des dérivés de la benzothiazépine comme ayant une activité anticalcique.

Dans la formule générale et dans ce qui suit, le terme atome d'halogène désigne, de préférence, un atome de chlore mais peut également représenter un atome de fluor, de brome ou d'iode. Le terme radical alkyle renfermant de 1 à 4 atomes de carbone désigne, de préférence, un radical méthyle, éthyle ou isopropyle mais peut aussi représenter un radical n-propyle, butyle, iso-butyle, sec-butyle ou tert-butyle.

Le terme radical alkyloxy renfermant de 1 à 4 atomes de carbone désigne, de préférence, un radical méthoxy, éthoxy ou n-propoxy, mais peut aussi représenter un radical iso-propoxy, n-butoxy, iso-butoxy, sec-butoxy ou tert-butoxy.

Le terme monoalkylamino désigne de préférence un radical monoalkylamino ayant de 1 à 4 atomes de carbone et en particulier le radical méthylamino, éthylamino ou isopropylamino, le terme dialkylamino désigne de préférence, un radical dialkylamino dont les radicaux alkyles, identiques ou différents comportent de 1 à 4 atomes de carbone en particulier le radical diméthylamino, éthylméthylamino, diéthylamino ou dipropylamino et diisopropylamino.

Le radical hétérocyclique saturé à cinq, six ou sept chaînons que $R_1$ et $R_2$ peuvent former avec l'atome d'azote auquel ils sont liés, hétérocycle comprenant éventuellement un autre hétéroatome choisi parmi les atomes d'azote, d'oxygène ou de soufre, désigne de préférence un radical pyrrolidinyle, imidazolidinyle, pyrazolidinyle, pipéridyle, pipérazinyle ou morpholinyle. Ces radicaux peuvent éventuellement être substitués sur un carbone de l'hétérocycle ou sur l'atome d'azote lorsque l'hétérocycle comporte un deuxième atome d'azote par un radical alkyle renfermant de 1 à 4 atomes de carbone ou par un radical aryle ou arylalkyle renfermant de 6 à 12 atomes de carbone éventuellement substitué par 1, 2 ou 3 radicaux choisis parmi les atomes d'halogènes, les radicaux alkyle ou alkyloxy renfermant de 1 à 4 atomes de carbone.

Le terme radical aryle désigne de préférence un radical phényle, le terme radical arylalkyle désigne de préférence un radical benzyle ou phénéthyle. Parmi les substituants des radicaux aryle ou arylalkyle, on préfère les atomes d'halogènes, les radicaux méthyle, éthyle, méthoxy, éthoxy ou propoxy.

La salification des produits peut être effectuée selon les méthodes usuelles : comme exemples d'acides minéraux, on peut citer les acides chlorhydrique, bromhydrique, nitrique, phosphorique ou sulfurique.

Comme exemples d'acides organiques, on peut mentionner : les acides formique, acétique, acrylique, butyrique, adipique, glutarique, crotonique, propionique, benzoïque, maléique, fumarique, succinique, tartrique, citrique, oxalique, glyoxylique, aspartique, les acides hexanoïques, heptanoïque, décanoïque, oléïque, stéarique, palmitique, ou leurs dérivés qui sont, par exemple, les acides isobutyrique, (4-méthyl pentanoïque), chloropropionique, phénylacétique, 2-thiophéneacétique, 3-thiophéneacétique, (4-éthyl phényl) acétique, l'ester monoéthylique de l'acide adipique, 3-hydroxypropionique, 3-méthoxy propionique, 3-méthylthio butyrique, 4-chloro butyrique, 4-phényl butyrique, 3-phénoxy butyrique, 4-éthyl benzoïque, 1-propyl benzoïque, ou encore les acides alcanesulfoniques tels que les acides méthane ou éthanesulfoniques, arènesulfoniques tels que l'acide benzènesulfonique.

L'invention a notamment pour objet les produits de formule générale (I), telle que définie ci-dessus, dans laquelle X représente un atome d'hydrogène ainsi que leurs sels d'addition avec les acides minéraux ou organiques.

L'invention a particulièrement pour objet les produits de formule générale (I), telle que définie précédemment, dans laquelle $R_1$ et $R_2$ représentent chacun un radical méthyle, éthyle, isopropyle, ceux dans laquelle $R_1$ représente un radical méthyle et $R_2$ représente un radical phénéthyle éventuellement mono ou di substitué par un radical méthyle ou méthoxy, ceux dans lesquels $R_1$ représente un atome d'hydrogène et $R_2$ représente un radical méthyle ou éthyle substitué par une dialkylamine, ou $R_1$ et $R_2$ forment avec l'atome d'azote auquel ils sont liés un radical pyrrolidinyle, pipéridyle, morpholinyle ou pipérazinyle éventuellement substitué sur l'atome d'azote non lié au radical $-(CH_2)_s-$ par un radical alkyle renfermant de 1 à 4 atomes de carbone ou par un radical aryle ou arylalkyle renfermant de 6 à 12 atomes de carbone lui-même éventuellement substitué par un atome d'halogène ou un radical alkyle ou alkyloxy renfermant de 1 à 4 atomes de carbone ainsi que leurs sels d'addition avec les acides minéraux ou organiques.

Lorsque $R_1$ et $R_2$ forment avec l'atome d'azote auquel ils sont liés, un radical pipérazinyle éventuellement substitué, on préfère les substituants méthyle ou phényle, eux-mêmes éventuellement substitués par un radical méthoxy.

L'invention a également pour objet, les produits de formule générale (I) telle que définie précédemment, dans laquelle $X_1$ et $X_2$ identiques ou différents représentent un atome de chlore ou de brome ou un radical hydroxy ou méthoxy et $X_3$ représente un atome d'hydrogène ainsi que leurs sels d'addition avec les acides minéraux ou organiques.

L'invention a plus particulièrement pour objet, les produits de formule générale (I), telle que définie précédemment dans laquelle Y représente le groupement

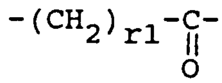

dans lequel r1 représente le nombre 1.

L'invention a également pour objet en particulier les produits de formule générale (I) telle que définie précédemment dans laquelle Y représente le groupement

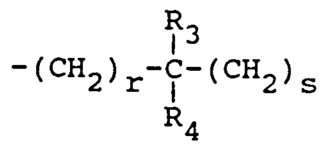

et parmi ceux-ci, ceux dans laquelle la somme r + s est égale à 1 ou 2, et $R_3$ et $R_4$ représentent chacun un atome d'hydrogène ainsi que leurs sels d'addition avec les acides, minéraux ou organiques et tout particulièrement :

- le (±)-5-[2-(diméthylamino) éthyl] 2,3-dihydro 3-(4-chloro phényl) 1,5-benzothiazépin-4(5H)-one,
- le (±)-5-[2-(diméthylamino) éthyl] 2,3-dihydro 3-(2-méthoxy phényl) 1,5-benzothiazépin-4(5H)-one,
- le (±)-5-[2-(diméthylamino) éthyl] 2,3-dihydro 3-(4-méthoxy phényl) 1,5-benzothiazépin-4(5H)-one, ainsi que leurs sels d'addition avec les acides minéraux ou organiques.

L'invention concerne aussi un procédé de préparation des produits de formule (I) telle que définie précédemment, caractérisé en ce que :

1) pour préparer des produits de formule (I) dans laquelle Y représente le groupe

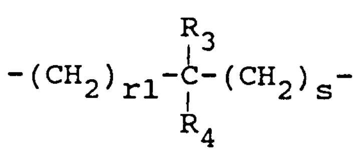

l'on traite un produit de formule (II) :

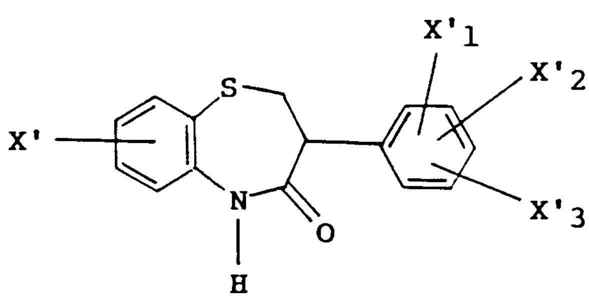

(II)

sous forme racémique ou optiquement active dans laquelle X' a la signification de X, telle que définie précédemment ou bien X' représente un radical réactif protégé, et $X'_1$, $X'_2$ et $X'_3$ identiques ou différents ont la signification de $X_1$, $X_2$ et $X_3$ telle que définie précédemment ou bien représentent un radical réactif protégé :

- soit par un produit de formule (III) :

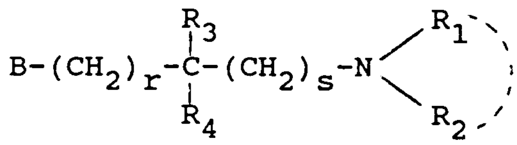

(III)

dans laquelle $R_1$, $R_2$, $R_3$, $R_4$, r et s ont les significations indiquées précédemment et B représente un atome d'halogène ou un radical hydroxyle, pour obtenir le produit de formule (X) :

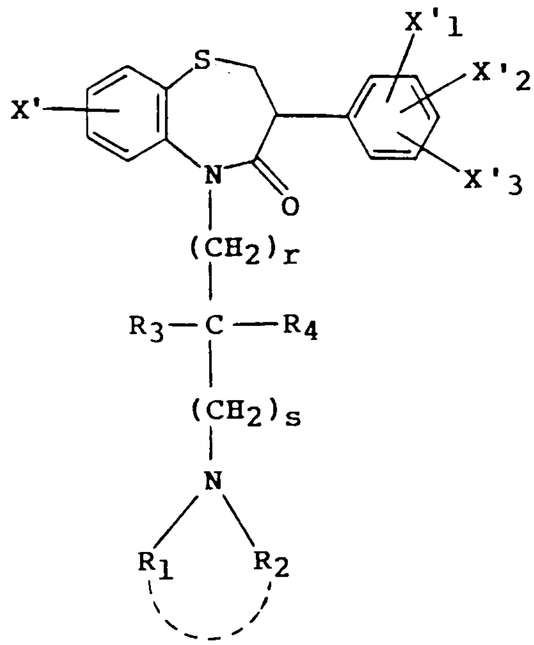

(X)

- soit, pour obtenir un produit de formule (I) dans laquelle s représente $s_1$, $s_1$ étant un entier de 1 à 4, par un produit de formule (IV) :

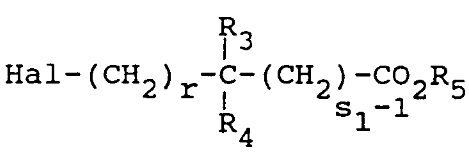

(IV)

dans laquelle Hal représente un atome d'halogène, $R_3$, $R_4$ et r ont les significations indiquées précédemment,

$s_1$ a la signification indiquée ci-dessus,

$R_5$ représente un radical alkyle renfermant de 1 à 4 atomes de carbone,

pour obtenir un produit de formule (V) :

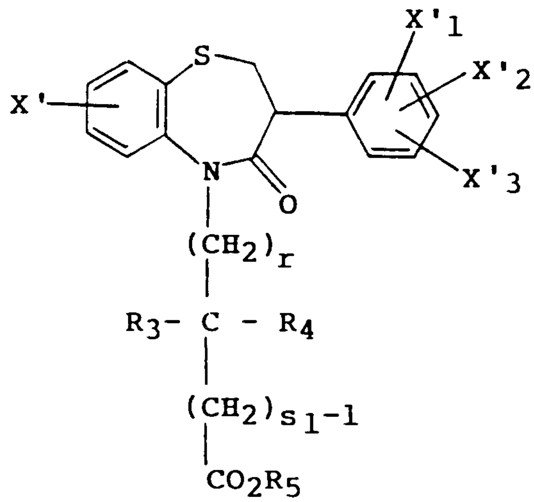

(V)

que l'on soumet à une réaction d'hydrolyse pour obtenir le composé de formule (VI) :

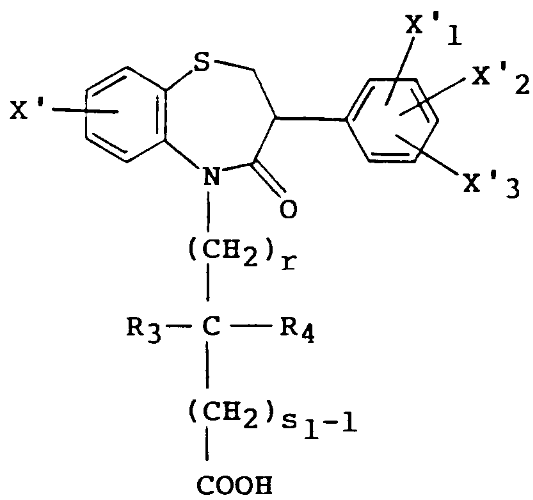

(VI)

que l'on soumet à une réaction de réduction pour obtenir un produit de formule (VII) :

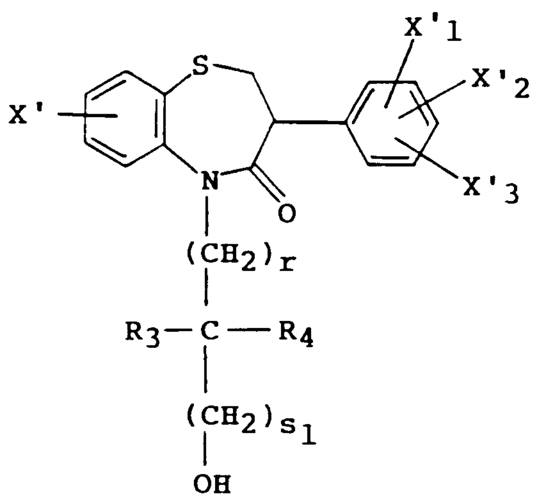

(VII)

que l'on soumet à une réaction de substitution par un atome d'halogène Hal pour obtenir un produit de formule (VIII) :

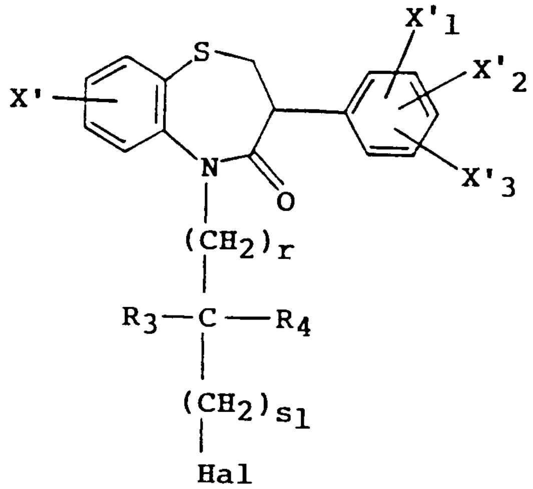

(VIII)

que l'on fait réagir avec l'amine de formule (IX) :

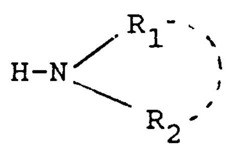

(IX)

pour obtenir un produit de formule (XI) :

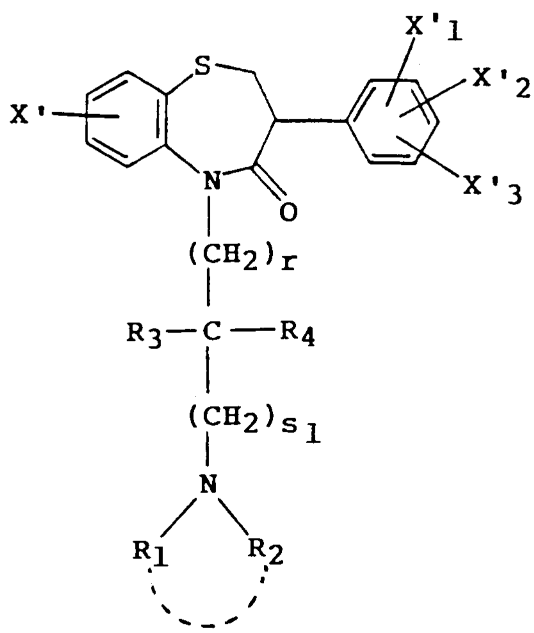

(XI)

2) pour préparer des produits de formule (I) dans laquelle Y représente un groupe

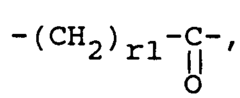

l'on traite un produit de formule (II) telle que définie ci-dessus soit par un produit de formule (III') :

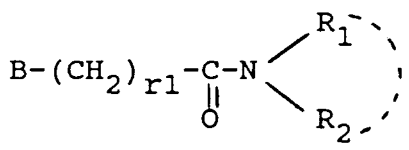

(III')

dans laquelle B, r1, $R_1$ et $R_2$ ont la signification indiquée précédemment pour obtenir un produit de formule (XVII) :

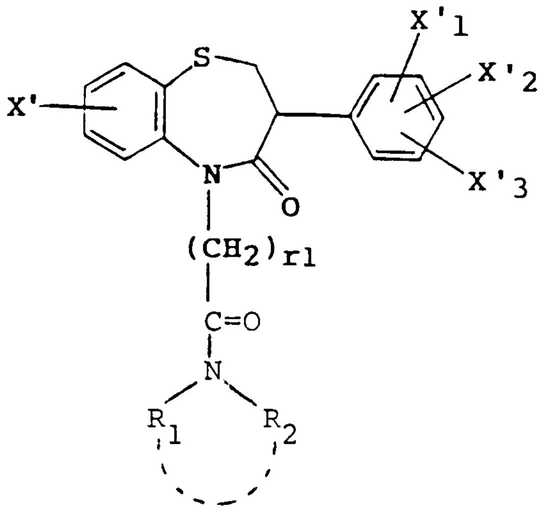

(XVII)

soit par un produit de formule (XVIII) :

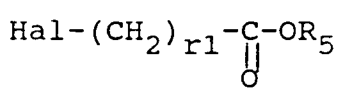

(XVIII)

dans laquelle $R_5$ et r1 ont les valeurs indiquées précédemment pour obtenir un produit de formule (XIX) :

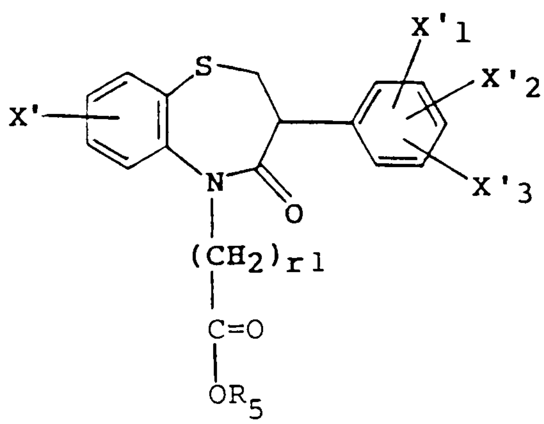

(XIX)

que l'on soumet à une réaction d'hydrolyse pour obtenir le composé de formule (XX) :

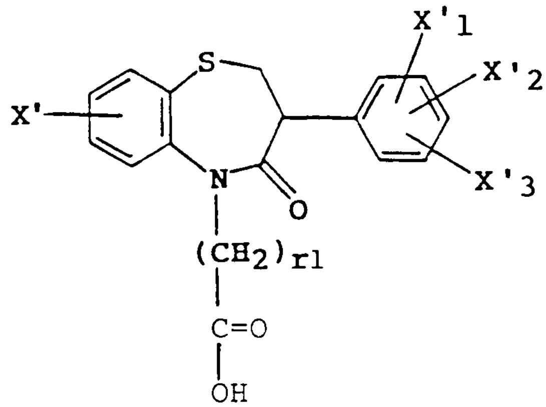

(XX)

que l'on fait réagir avec une amine de formule (IX) telle que définie ci-dessus pour obtenir un produit de formule (XVII) telle que définie ci-dessus, puis soumet si nécessaire ou si désiré les produits de formule (X), (XI) et (XVII) obtenus sous forme racémique ou optiquement active, à l'une ou plusieurs des réactions suivantes, dans un ordre quelconque :

a) coupure par hydrolyse ou hydrogénolyse du groupe protecteur du ou des radicaux réactifs protégés,

b) dédoublement des produits racémiques par des procédés classiques pour obtenir les produits optiquement actifs,

c) salification pour obtenir les sels correspondants.

Le groupe protecteur du radical réactif amino ou monoalkylamino protégé que peut représenter X', $X'_1$, $X'_2$ ou $X'_3$, peut être par exemple :

- un radical alkyle de 4 à 6 atomes de carbone tel que, préférentiellement, tert-butyle ou tert-pentyle,
- un groupe acyle, aliphatique, aromatique ou hétérocyclique ou un groupe carbamoyle. On peut citer les groupes alcanoyles inférieurs tels que par exemple formyle, acétyle, propionyle, butyryle, benzoyle, isobutyryle, valéryle, isovaléryle, oxalyle, succinyle, pivaloyle, les groupes acyles substitués tels que chloroacétyle,
- un groupe alkyloxy ou cycloalkyloxycarbonyle inférieur tel que par exemple, méthoxycarbonyle, éthoxycarbonyle, propoxycarbonyle, (1-cyclopropyl éthoxycarbonyle), isopropyloxycarbonyle, butyloxycarbonyle, tert-butyloxycarbonyle, pentyloxycarbonyle, hexyloxycarbonyle,
- un groupe arylalkyle inférieur tel que benzyle, 4-méthoxy benzyle ou phényléthyle, trityle, (3,4-diméthoxy benzyle) ou benzhydryle,
- un groupe haloalkyle tel que trichloroéthyle,
- un groupe chlorobenzoyle, para-nitro benzoyle, para ou tert-butyl benzoyle, phénoxyacétyle, décanoyle, acryloyle, trichloro éthoxycarbonyle,
- un groupe méthylcarbamoyle, phénylcarbamoyle, naphtylcarbamoyle, ainsi que les thiocarbamoyles correspondants.

La liste ci-dessus n'est pas limitative, il est évident que d'autres groupes protecteurs des amines, groupes connus en particulier dans la chimie des peptides, peuvent également être utilisés.

Le groupe protecteur du radical hydroxyle protégé que peut représenter X', $X'_1$, $X'_2$ ou $X'_3$, peut être choisi dans la liste ci-dessous :

- un groupe acyle tel que par exemple formyle, acétyle, chloroacétyle, bromoacétyle, dichloroacétyle, trichloroacétyle, trifluoroacétyle, méthoxyacétyle, phénoxyacétyle, benzoyle, benzoylformyle, p-nitro benzoyle. On peut citer également les groupes éthoxycarbonyle, méthoxycarbonyle, propoxycarbonyle, (2,2,2-trichloro éthoxy) carbonyle, benzyloxycarbonyle, tert-butoxycarbonyle, (1-cyclopropyl éthoxy) carbonyle, tétrahydropyrannyle, tétrahydrothiopyrannyle, méthoxytétrahydropyrannyle, trityle, benzyle, 4-méthoxy benzyle, benzhydryle, trichloroéthyle, (1-méthyl 1-méthoxy éthyle), phtaloyle.

On peut également citer d'autres acyles tels que propionyle, butyryle, isobutyryle, valéryle, isovaléryle, oxalyle, succinyle et pivaloyle.

On peut également citer les radicaux phénylacétyle, phénylpropionyle, mésyle, chlorobenzoyle, para-nitrobenzoyle, para-(tert-butyl) benzoyle, acryloyle, méthyl-carbamoyle, phénylcarbamoyle.

Dans un mode de réalisation préférée du procédé de l'invention :

- dans le produit de formule (III) B est un hydroxyle ou un atome de chlore,
- dans le produit de formule (III') B est de préférence un atome de brome ou de chlore,

- la réaction d'addition du composé de formule (III) sur le composé de formule (II) peut s'effectuer :
  . dans le cas où B est un hydroxyle, en présence d'azodicarboxylate de diéthyle, de triphénylphosphine, de dicyclohexylcarbodiimide et en particulier dans un éther qui est de préférence l'éther éthylique ou encore le tétrahydrofuranne ou le dioxanne. La réaction se fait en un temps variable de 4 à 24 heures à la température ambiante,
  . dans le cas où B est un halogène, en présence d'une base qui peut être la soude ou de préférence l'hydrure de sodium, dans du diméthylformamide pendant un temps de l'ordre de 4 heures à une température de l'ordre de 60 ° C.
- dans le composé de formule (IV) ou (XVIII), l'atome d'halogène peut être un atome de brome mais également de chlore, d'iode ou de fluor.
- la réaction d'addition du composé de formule (IV) ou (XVIII) sur le composé de formule (II) s'effectue en présence d'une base minérale de préférence l'hydrure de sodium.
- l'hydrolyse de l'ester de formule (V) ou (XIX) en l'acide de formule (VI) ou (XX) s'effectue en milieu acide préférentiellement l'acide sulfurique aqueux en présence d'acide acétique mais encore d'acide chlorhydrique ou bromhydrique aqueux.
- la réduction sélective de l'acide de formule (VI) en l'alcool de formule (VII) s'effectue préférentiellement en présence d'un complexe du diborane ou encore d'hydrure d'aluminium et de lithium ou d'hydrure de diisobutylaluminium.
- la préparation du dérivé halogéné de formule (VIII) se fait avantageusement par action de chlorure de thionyle dans du dichlorométhane pendant une durée de quelques minutes à la température ambiante.
- l'addition de l'amine de formule (IX) sur les produits de formule (VIII) ou (XX) se fait indifféremment avec ou sans solvant organique à la température ambiante ou à chaud.

Selon les valeurs de X′, $X'_1$, $X'_2$ et $X'_3$, les produits de formule (X), (XI) et (XVII) peuvent ou non constituer des produits de formule (I).

Les produits de formule (X), (XI) et (XVII) constituent des produits de formule (I) lorsque X′, $X'_1$, $X'_2$ et/ou $X'_3$ ne représentent pas un radical réactif protégé, ce radical réactif étant hydroxyle, amino ou monoalkylamino.

Dans les autres cas, l'action sur le produit de formule (X), (XI) ou (XVII) d'un ou plusieurs agents d'hydrolyse, d'hydrogénolyse ou de la thiourée a pour but d'éliminer le groupe protecteur dans X′, $X'_1$, $X'_2$ ou $X'_3$ quand celui-ci protège un radical hydroxyle, amino ou monoalkylamino.

La nature des réactifs à mettre en jeu dans un tel cas, est bien connue de l'homme de métier. On donne ci-après une énumération non exhaustive des moyens pouvant être mis en oeuvre pour éliminer les différents groupes.

L'élimination du groupe protecteur de X′, $X'_1$, $X'_2$ et/ou $X'_3$, quand la fonction protégée est un radical amino ou monoalkylamino hydroxyle, peut être effectuée par hydrolyse, celle-ci étant acide.

L'acide que l'on utilise de préférence peut être choisi dans le group constitué par les acides chlorhydrique, benzène sulfonique ou para-toluène sulfonique, formique ou trifluoroacétique. On peut cependant utiliser d'autres acides minéraux ou organiques.

Les groupements tels que trichloroéthyle, benzhydryle, benzyloxycarbonyle, sont éliminés de préférence par hydrogénolyse. On peut citer le système zinc-acide acétique ou l'hydrogène en présence d'un catalyseur.

L'élimination du groupe chloroacétyle peut être effectuée par la thiourée, par exemple, selon les conditions décrites dans MASAKI JACS 90 4508 (1968).

Des indications sur les groupes protecteurs et leurs méthodes d'élimination sont également données, par exemple, dans le brevet français FR-A-2.499.995.

Naturellement on peut, lorsque par exemple X′, $X'_1$, $X'_2$ et $X'_3$ sont des groupes éliminables appartenant à des types différents, faire agir sur les produits plusieurs agents envisagés dans les énumérations précédentes.

La salification des produits peut être effectuée selon les méthodes usuelles en utilisant un des acides minéraux ou organiques choisis dans la liste indiquée ci-dessus.

Les composés de formule (I) tels que définis ci-dessus, ainsi que leurs sels d'addition avec les acides présentent d'intéressantes propriétés pharmacologiques. Ils présentent en particulier des propriétés anti-arythmiques, anti-agrégantes plaquettaires et anti-sérotoninergiques.

Certains des composés de formule (I) présentent en outre une activité anti-calcique.

Les propriétés anti-arythmiques des composés objets de l'invention, ainsi que leurs sels d'addition avec les acides minéraux ou organiques pharmaceutiquement acceptables permettent notamment de traiter les troubles du rythme cardiaque. Leur effet anti-calcique associé à leurs propriétés anti-agrégantes plaquettaires et anti-sérotoninergiques les rendent actifs dans le traitement de l'angine de poitrine aussi bien dans le cas d'angor spastique que d'angor instable.

Ces propriétés anti-agrégantes plaquettaires et anti-sérotoninergiques, leur font également trouver leur emploi dans le traitement des crises migraineuses ou encore dans un but préventif anti-thrombotique.

L'invention a également pour objet à titre de médicaments, les produits de formule (I) tels que définis ci-dessus, ainsi que leurs sels d'addition avec les acides minéraux ou organiques pharmaceutiquement acceptables.

La présente invention a tout particulièrement pour objet, à titre de médicaments :

- le (±)-5-[2-(diméthylamino) éthyl] 2,3-dihydro 3-(4-chlorophényl) 1,5-benzothiazépin-4(5H)-one,
- le (±)-5-[2-(diméthylamino) éthyl] 2,3-dihydro 3-(2-méthoxyphényl) 1,5-benzothiazépin-4(5H)-one,
- le (±)-5-[2-(diméthylamino) éthyl] 2,3-dihydro 3-(4-méthoxyphényl) 1,5-benzothiazépin-4(5H)-one, ainsi que leurs sels d'addition avec les acides minéraux ou organiques pharmaceutiquement acceptables.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 et 400 mg de principe actif, par voie orale et entre 5 et 100 mg par jour, par voie parentérale.

L'invention comprend de plus à titre de produits industriels nouveaux, les produits intermédiaires répondant aux formules (V), (VI), (VII), (VIII), (XIX) et (XX) tels que définis ci-dessus.

Les produits de formule (II) parmi lesquels les 3-(2,4-dichlorophényl) 2,3-dihydro 1,5-benzothiazépin-4-(5)-one et 3-(2,5-dichlorophényl) sont connus (J. Med. Chem. 8 (3) 511 (1971) et peuvent être préparés à partir des produits de formule (XII) :

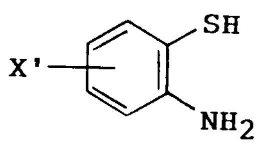

(XII)

et des produits de formule (XIII) :

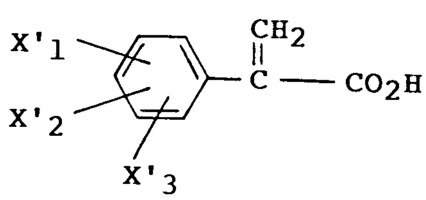

(XIII)

Les produits de formule (XII) sont eux-mêmes connus ou peuvent eux-mêmes être préparés selon les méthodes usuelles par exemple celles décrites dans les références suivantes :

- LIEBIGS Ann. Chem. 1987, (11) 921-5,
- V.A.R.J. Chem. 1971, 14 (5), 493-505,
- Demande de brevet allemande DE 26.38.760,
- Demande de brevet japonaise 79/145.678.

Les produits de formule (XIII) sont connus et peuvent être préparés selon la méthode décrite ci-dessous dans la partie expérimentale et dans le brevet USP 3.574.215 qui consiste à faire agir du diéthyloxalate

dans le toluène en présence d'éthylate de sodium, sur un produit de formule (XIV) :

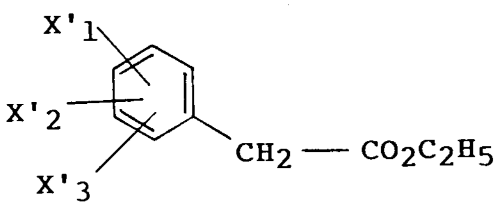

(XIV)

pour obtenir un produit de formule (XV) :

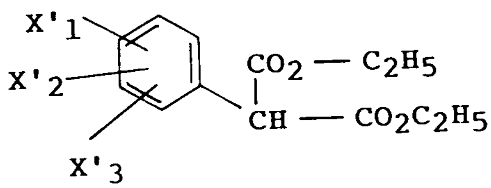

(XV)

que l'on soumet à la formaldéhyde en présence de $K_2CO_3$ pour obtenir le produit de formule (XVI) :

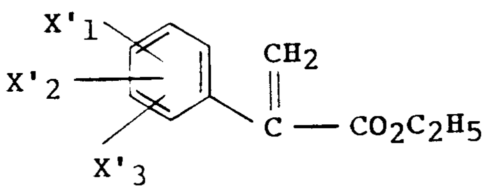

(XVI)

qui soumis à une réaction de saponification par action de la potasse en milieu THF-$H_2O$, permet d'obtenir le produit de formule (XIII). Les produits de formule (XIII) peuvent encore être préparés suivant l'un des deux techniques décrites respectivement dans les articles EUR. J. MED. CHEM 1979 14 (3) 207-214 et J. MED. CHEM. 1969 12 (3) 477-480. Certains des produits de formule (III), (III') et (IV) sont connus ou même accessibles dans le commerce, c'est notamment le cas des produits utilisés dans les exemples décrits ci-après. Les produits de formule (III) qui ne sont pas décrits peuvent être préparés selon les méthodes connues de l'homme de métier.

Des exemples de préparation de produits de formule (III) sont indiqués dans les publications suivantes :

- Beil 37, 3508 (1904) ou J. Chem. Soc. 1927, 1012, cités dans le Merck Index 10ème édition n° 2825,
- Beil 20 (2) 5.

Des exemples de préparation de produits de formule (IV) sont donnés dans les articles référencés comme suit :

- "I of PHARMACY and PHARMACOLOGY", 1952, II, p. 61.
- Beil 2, 325,
- Beil 2 (3) 737.

Des exemples d'une telle préparation sont donnés ci-après dans la partie expérimentale.

Les exemples qui suivent illustrent la présente invention.

**<u>EXEMPLE 1</u> : (Z)-2-butènedioate de 2,3-dihydro 3-(4-méthoxy phényl) 5-[2-(1-pyrrolidinyl) éthyl] 1,5-benzothiazépin-4(5H)-one.**

<u>STADE A</u> : 2,3-dihydro 3-(4-méthoxy phényl) 5-[2-(1-pyrrolidinyl) éthyl] 1,5-benzothiazépin-4(5H)-one.

A une solution de 2,85 g de produit obtenu au stade de la préparation 1 et 5,25 g de triphényl phosphine, dans 100 $cm^3$ de tétrahydrofuranne anhydre, on ajoute 2,3 g de 1-(2-hydroxy éthyl) pyrrolidine puis, en 10 minutes, 3,48 g d'azodicarboxylate de diéthyle en solution dans 50 $cm^3$ de tétrahydrofuranne anhydre. On agite pendant 22 heures à température ambiante, évapore à sec sous pression réduite et

obtient 15 g de produit recherché brut que l'on chromatographie sur silice, on recueille 2,61 g de produit attendu.

Spectre RMN (250 MHz, $CDCl_3$)

| | |
|---|---|
| 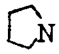 | 1,78 (m), 2,66 (m) |
| $C\underline{H}_2$-N | 2,45 à 3,00 (m) |
| O-$C\underline{H}_3$ | 3,77 (s) |
| $C\underline{H}_2$-$C\underline{H}$ (3H) | de 3,40 à 4,50 (m) |
| 2H en ortho de O-$CH_3$ | 6,82 (d) |
| 2H en méta de O-$CH_3$ | 7,25 (d) |
| 3 autres H aromatiques | 7,10 à 7,60 (m) |
| H6 base d'intégration | 7,66 (d, l) |

STADE B : (Z)-2-butènedioate de 2,3-dihydro 3-(4-méthoxy phényl) 5-[2-(1-pyrrolidinyl) éthyl] 1,5-benzothiazépin-4(5H)-one.

A une solution de 2,26 g de produit obtenu au stade A, dans 15 $cm^3$ d'isopropanol, on ajoute une solution d'acide maléique en quantité stoechiométrique dans 10 $cm^3$ d'isopropanol. On concentre à un volume total de 15 $cm^3$, laisse au repos 8 heures à température ambiante, 1 nuit à 0°C, essore à température ambiante, lave avec 3 $cm^3$ d'isopropanol et 10 $cm^3$ d'éther éthylique. On obtient 1,41 g de produit F = 170°C. On dissout 1,4 g de ce dernier dans 150 $cm^3$ d'isopropanol à reflux, concentre à 20 $cm^3$, laisse en repos 20 heures à température ambiante et essore. On obtient 1,29 g de produit recherché, F = 170°C.

| Analyse pour $C_{26}H_{30}N_2O_6S$ = 498,6 | | | | | | | | |
|---|---|---|---|---|---|---|---|---|
| Calculés<br>Trouvés | C% | 62,63<br>62,5 | H% | 6,06<br>6,3 | N% | 5,62<br>5,5 | S% | 6,43<br>6,2 |

Spectre RMN ($CDCl_3$, 250 MHz)

2,08 H en béta de N pyrrolidine

| | |
|---|---|
| 3,78 (s) | $O-CH_3$ |
| de 2,7 à 4,5 (m) | $NCH_2-CH_2-N$ |
| | **CH** en alpha de N pyrrolidine |
| | $SCH_2-CH-(C_6H_5)$ |
| 6,23 (s) | éthyléniques |
| 6,85 (d) | H en ortho de $O-CH_3$ |
| 7,21 (d) | H en méta de $O-CH_3$ |
| 7,83 (t) | les autres aromatiques |
| 7,41 (d) | |
| 7,57 (t) | |
| 7,68 (d) | |

**Préparation 1** : 2,3-dihydro 3-(4-méthoxy phényl) 1,5-benzothiazépin-4(5H)-one.

STADE A : 4-méthoxy alpha-méthylène benzèneacétate de méthyle.

A une suspension composée de 13,8 g de méthylate de sodium (préparé à partir de 5,9 g de sodium) et 400 $cm^3$ d'éther éthylique on ajoute en 15 minutes une solution de 36 g de méthyl 4-méthoxy phénylacétate, 32 g de diméthyl oxalate et 250 $cm^3$ d'éther éthylique. On agite 17 heures à reflux. On ajoute 200 $cm^3$ d'éther puis, en refroidissant, ajoute en 30 minutes 200 $cm^3$ d'acide chlorhydrique 2N. On décante, lave la phase éthérée avec 50 $cm^3$ d'acide chlorhydrique 2N puis 3 fois 50 $cm^3$ d'une solution saturée de chlorure de sodium et amène à sec sous pression réduite. Le résidu est repris par 400 $cm^3$ d'eau et on ajoute, en présence d'une trace d'hydroquinone, 40 $cm^3$ d'aldéhyde formique à 40 % et 30,4 g de carbonate de potassium. On agite pendant 3 heures à température ambiante. Après extraction avec de l'acétate d'éthyle et lavage à l'eau, on amène à sec sous pression réduite. On chromatographie le résidu sur silice (éluant cyclohexaneacétate d'éthyle : 95-5). On obtient 26 g de produit recherché.

Spectre RMN ($CDCl_3$) 60 MHz

| | |
|---|---|
| Les $OCH_3$ | 227 Hz |
| $(C_6H_5)-C = C-H$ | 348 (d, j ≃ 1,5 Hz) |
| $CO_2$ H | 375 (d, j ≃ 1,5 Hz) |
| Aromatiques | 408 à 446 Hz. |

STADE B : Acide 4-méthoxy alpha-méthylène benzèneacétique.

A une solution de 26 g du produit obtenu au stade A, dans 750 $cm^3$ de tétrahydrofuranne, on ajoute 500 $cm^3$ d'une solution aqueuse de potasse à 25 % et agite pendant 66 heures à température ambiante. Après évaporation du solvant, on ajoute 500 $cm^3$ d'eau et lave la phase aqueuse avec de l'acétate d'éthyle puis l'acidifie avec de l'acide chlorhydrique concentré. On obtient après essorage 23 g de produit recherché, F = 118 °C, utilisé tel quel pour le stade suivant.

Un échantillon analytique a été obtenu par recristallisation de 2 g du produit brut dans 150 $cm^3$ d'eau. On recueille 1,1 g de produit purifié. F = 118 °C.

Spectre IR ($CHCl_3$ sur Nicolet)

-C(=O)- 1727 $cm^{-1}$ (ep.)

1697 $cm^{-1}$ (max.)

- C = C 1612 $cm^{-1}$ (F)

\+ 1575 $cm^{-1}$

aromatiques 1513 $cm^{-1}$ (F).

STADE C : Acide alpha-[[(2-amino phényl) thio] méthyl] 4-méthoxy benzèneacétique.

On chauffe à reflux pendant 7 heures une solution composée de 125 $cm^3$ d'éthanol, 8,9 g d'acide 4-méthoxy alphaméthylène benzèneacétique et 5,35 $cm^3$ de 2-amino thiophénol. On évapore l'éthanol et cristallise le résidu dans 500 $cm^3$ d'éther isopropylique. On obtient 11 g de produit recherché. F = 118 °C, utilisé tel quel pour le stade suivant.

Un échantillon analytique a été préparé par recristallisation de 200 mg de produit brut dans 20 $cm^3$ d'éther isopropylique, on obtient 140 mg de produit purifié. F = 118 °C.

Spectre RMN ($CDCl_3$, 60 MHz)

= C-OC$H_3$ 3 H ≃ 225 Hz

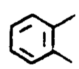 ≃ 4 H 387 à 414 Hz

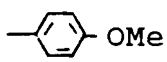 4 H 417 à 443 Hz

Les H mobiles ≃ 3 H 364 Hz

Les autres H 168 à 227 Hz

STADE D : 2,3-dihydro 3-(4-méthoxy phényl) 1,5-benzothiazépin-4(5H)-one.

A une solution de 10,3 g de produit obtenu au stade C, dans 300 $cm^3$ d'éthanol, on ajoute, en refroidissant, 7 g de dicyclohexylcarbodiimide. On agite pendant 17 heures à température ambiante, évapore l'éthanol et reprend le résidu par 150 $cm^3$ d'acétate d'éthyle. On filtre l'insoluble et évapore le filtrat à sec sous pression réduite. On reprend le résidu par 25 $cm^3$ d'isopropanol et obtient après essorage 5,85 g de produit recherché F = 145 °C. Le filtrat est chromatographié sur silice (éluant cyclohexane-acétate d'éthyle : 5-5). On recueille ainsi encore 2,2 g de produit après cristallisation dans 20 $cm^3$ d'isopropanol. F = 146 °C.

| Analyse pour $C_{16}H_{15}NO_2S$ = 285,352 | | | | | | | | |
|---|---|---|---|---|---|---|---|---|
| Calculés<br>Trouvés | C% | 67,34<br>67,1 | H% | 5,30<br>5,2 | N% | 4,91<br>4,7 | S% | 11,23<br>11,1 |

Spectre IR ($CHCl_3$ sur Nicolet)

-NH-C(=O)- { 3387 $cm^{-1}$ ; 1682 $cm^{-1}$ }

-OMe 2838 $cm^{-1}$

| Spectre RMN ($CDCl_3$, 90 MHz) | |
|---|---|
| S-C**H**$_2$-C**H** | 203 à 243 Hz |
| -OC**H**$_3$ | 226 Hz |
| N**H** | 478 Hz |
| Les **H** aromatiques | 406 à 465 Hz. |

**EXEMPLE 2 : Chlorhydrate de (±)-3-(3-bromo 4-méthoxy phényl)-2,3-dihydro 5-[2-(diméthylamino) éthyl] 1,5-benzothiazépin-4(5H)-one.**

STADE A : 3-(3-bromo 4-méthoxy phényl) 2,3-dihydro 1,5-benzothiazépin-4(5H)-one.

A une solution de 2,28 g de 2,3-dihydro 3-(4-méthoxy phényl) 1,5-benzothiazépin-4(5H)-one, obtenue au stade D de la préparation 1, dans 16 $cm^3$ d'acide acétique, on ajoute 24 $cm^3$ d'une solution N de brome dans l'acide acétique. Après 22 heures d'agitation à température ambiante on ajoute 100 $cm^3$ d'essence G, sépare le précipité gommeux obtenu en décantant la solution puis cristallise celui-ci dans 20 $cm^3$ d'éthanol. On agite 30 minutes et essore. On obtient 2,22 g de produit recherché F = 200°C puis 220°C, le produit est utilisé tel quel pour le stade suivant.

On a prépare un échantillon analytique en dissolvant 540 mg du produit ci-dessus dans 20 $cm^3$ de chlorure de méthylène. On traite avec du charbon actif, filtre, ajoute 50 $cm^3$ d'éthanol et concentre à 25 $cm^3$. Après essorage, on obtient 450 mg de produit F = 206°C puis 220°C. On redissout ce produit dans 60 $cm^3$ d'éthanol à reflux, concentre à 40 $cm^3$ et essore après 1 nuit à température ambiante. On obtient 350 mg de produit F = 210° puis 220°C.

| Analyse pour $C_{16}H_{14}BrNO_2S$ = 364,28 | | | | | | | | | | |
|---|---|---|---|---|---|---|---|---|---|---|
| Calculés | C% | 52,75 | H% | 3,87 | Br% | 21,94 | N% | 3,85 | S% | 8,80 |
| Trouvés | | 52,4 | | 3,8 | | 22,0 | | 3,9 | | 8,8 |

| Spectre RMN (DMSO, 250 MHz) | |
|---|---|
| S-C**H**$_2$-CH 2H | de 3,46 à 3,65 |
| H en ortho de O-$CH_3$ | 7,0 (d) |
| H en ortho de Br | 7,57 (d, j = $2H_3$) |
| -OC**H**$_3$ | 3,81 |
| N-**H** | 9,97 (s) |
| Les autres aromatiques | 7,60 (d,d) 1H |
| | 7,44 (t) 1H |
| | 7,14 à 7,32 (m) 3H. |

STADE B : Chlorhydrate de (±)-3-(3-bromo 4-méthoxy phényl) 2,3-dihydro 5-[2-(diméthylamino) éthyl] 1,5-benzothiazépin4(5H)-one.

A une solution de 1,82 g de produit obtenu au stade A, dans 18,2 $cm^3$ de tétrahydrofuranne, on ajoute en une seule fois 669 mg de diméthylamino éthanol en solution dans 18,2 $cm^3$ de tétrahydrofuranne, puis 1,97 g de triphényl phosphine. On glace puis ajoute une solution de 1,31 g d'azodicarboxylate de diéthyle dans 18,2 $cm^3$ de tétrahydrofuranne et agite pendant 19 heures à température ambiante. On évapore le tétrahydrofuranne sous pression réduite, extrait avec 3 fois 50 $cm^3$ d'acide chlorhydrique N, lave les extraits acides avec 3 fois 50 $cm^3$ d'acétate d'éthyle, alcalinise la fraction acide avec de la lessive de soude, extrait avec du chlorure de méthylène, lave à l'eau et concentre à sec sous pression réduite. On obtient 2 g de résidu que l'on chromatographie sur silice (éluant : chlorure de méthylène-méthanol : 9-1). On obtient 1 g de produit recherché sous forme de base.

Préparation du chlorhydrate :

A une solution de 1 g de la base ci-dessus, dans 30 $cm^3$ d'acétate d'éthyle et 1 $cm^3$ d'acétone, on ajoute une solution d'acétate d'éthyle chlorhydrique en léger excès. On obtient 1 g de chlorhydrate recherché. F = 150 °C.

On dissout le chlorhydrate obtenu, dans 150 $cm^3$ d'acétone à reflux, filtre, concentre à 20 $cm^3$. On obtient 770 mg de produit F = 152 °C, que l'on recristallise à nouveau par dissolution dans 100 $cm^3$ d'acétone au reflux et ajout à 25 °C de 100 $cm^3$ d'éther. On obtient 600 mg de produit recherché. F = 152 °C.

Spectre RMN ($CDCl_3$, 400 MHz)

| | | |
|---|---|---|
| 2,83 ppm (sl) | | les N-C**H3** |
| 3,12 (m) | 1H | |
| 3,32 à 3,51 (m) | 3H | S-C**H**$_2$-C**H** |
| 3,73 (d,d) | 1H | et |
| 4,18 (m) | 1H | N- |
| 4,65 (m) | 1H | C**H**$_2$-C**H**$_2$-N |
| 3,87 (s) | | |
| 6,85 (d) | | H en ortho de $OCH_3$ |
| 7,23 (d,d) | | H en para de Br |
| 7,47 (d) | | H en ortho de Br |

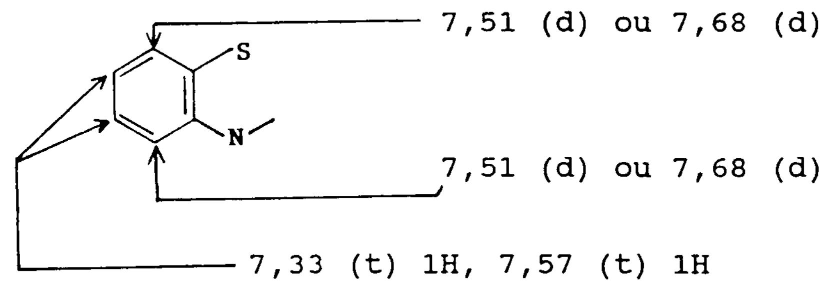


**EXEMPLE 3 : (Z)-2-butènedioate de 2,3-dihydro 3-(4-méthoxy phényl) 5-[2-(4-morpholinyl) éthyl] 1,5-benzothiazépin-4(5H)-one.**

A une solution de 1,97 g de triphényl phosphine dans 50 $cm^3$ de tétrahydrofuranne, on ajoute, à température ambiante, une solution de 1,3 g d'azodicarboxylate de diéthyle dans 20 $cm^3$ de tétrahydrofuranne. On agite 10 minutes et ajoute 1,43 g du produit obtenu au stade D de la préparation 1 et ensuite, 1 g de morpholino éthanol. On agite 24 heures à température ambiante. On évapore le tétrahydrofuranne et reprend le réside par 75 $cm^3$ d'acétate d'éthyle, extrait 3 fois 75 $cm^3$ d'acide chlorhydrique N, lave par 2 fois 75 $cm^3$ d'acétate d'éthyle et alcalinise à pH 10, en ajoutant de la lessive de soude. On extrait par 3 fois 100 $cm^3$ d'acétate d'éthyle, lave à l'eau, sèche, évapore à sec. On obtient 1,7 g de produit que l'on chromatographie sur silice (éluant : acétate d'éthyletriéthylamine : 95-5). On obtient 950 mg de produit auxquels on joint 1,34 g de produit obtenu de façon identique. On chromatographie 2,29 g sur silice (éluant : cyclohexane-dioxane : 70-30) et on obtient 1,6 g de produit attendu sous forme de base.

Salification.

On dissout 1,6 g de la base obtenue ci-dessus, dans 20 $cm^3$ d'isopropanol à 40 °C. A cette solution, on ajoute une solution de 0,460 g d'acide maléique dans 20 $cm^3$ d'isopropanol. On laisse en repos 16 heures. On essore, lave avec 5 $cm^3$ d'isopropanol et 10 $cm^3$ d'éther éthylique. On obtient 1,2 g de produit recherche. F = 176 °C.

Par recristallisation dans l'acétonitrile, on obtient 780 mg de produit. F = 178 ° C.

| Analyse pour $C_{26}H_{30}N_2O_7S$ = 514,58 | | | | | | | | |
|---|---|---|---|---|---|---|---|---|
| Calculés<br>Trouvés | C% | 60,68<br>60,4 | H% | 5,88<br>5,9 | N% | 5,45<br>5,5 | S% | 6,23<br>6,1 |

| Spectre RMN (250 MHz, $CDCl_3$) | |
|---|---|
| 3,78 (s) | O-C**H**$_3$ |
| de 3,00 à 4,05 (m) | ≃ 14 H |
| 4,49 (m) | NCO-C**H**-($C_6H_5$) |
| 6,85 (d,j = 8,5) | 2H en ortho de O-$CH_3$ |
| 7,21 (d,j = 8,5) | 2H en méta de O-$CH_3$ |
| de 7,30 à 7,72 (m) | 4H aromatiques |
| 6,24 (S) | $CO_2$H-C**H**=C**H**-$CO_2$H. |

**EXEMPLE 4 : (E)-2-butènediote de 2,3-dihydro 3-(4-méthoxy phényl) 5-[2-(1-piperidinyl) éthyl] 1,5-benzothiazépin-4(5H)-one.**

On opère comme à l'exemple 3 à partir de 4,29 g de produit obtenu au stade D de la préparation 1 avec 5,91 g de triphényl phosphine, 3,9 g d'azodicarboxylate d'éthyle et 2,91 g de piperidinethanol. On obtient 5,91 g d'extrait sec que l'on chromatographie sur silice (éluant chlorure de méthylène-méthanol : 95-5). On obtient 1,15 g de produit que l'on cristallise dans 3 $cm^3$ d'acétate d'éthyle. On obtient 270 mg de produit attendu sous forme de base. F = 110 ° C.

Salification :

A une solution de 640 mg de produit obtenu comme ci-dessus, dans 20 $cm^3$ d'isopropanol à 40 ° C, on ajoute 187 mg d'acide fumarique en solution dans 5 $cm^3$ de méthanol. On concentre jusqu'à 3 $cm^3$ et ajoute 5 $cm^3$ d'acétate d'éthyle. On essore et obtient 770 mg de produit attendu F = 174 ° C. On effectue une seconde purification en dissolvant le produit ci-dessus dans 5 $cm^3$ de méthanol à reflux. On ajoute 30 $cm^3$ de méthyl éthyl cétone, concentre à 5 $cm^3$, essore après 20 H à 0 ° C. On obtient 680 mg de produit recherché.

| Analyse pour $C_{27}H_{32}N_2O_6S$ = 512,607 | | | | | | | | |
|---|---|---|---|---|---|---|---|---|
| Calculés<br>Trouvés | C% | 63,26<br>63,1 | H% | 6,29<br>6,3 | N% | 5,46<br>5,4 | S% | 6,25<br>6,2 |

Spectre RMN (250 MHz, $CDCl_3$)

1,57 (m) pour a

1,83 (m) pour b et c 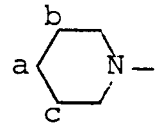

$C\underline{H}_2-S(C_6H_5)$

de 3,0 à 3,5 (m) 8H et 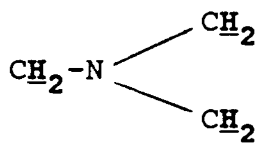

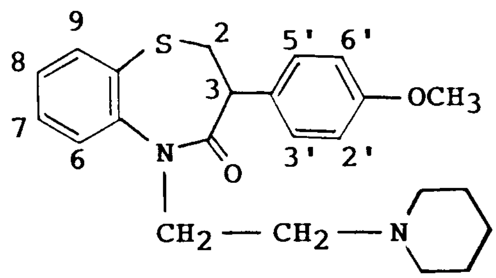

| | |
|---|---|
| 3,76 (dd,j = 12,5 et 6,5), | Hz |
| 3,77 (s) | $OC\underline{H}_3$ |
| 4,06 (m) | $C\underline{H}_2$-NCO |
| 4,42 (m) | |
| 6,84 (d, j = 8,5) | $H_2$' et $H_6$' |
| 7,21 (d, " ) | $H_3$' et $H_5$' |
| 7,27 (dl) | $H_9$ |
| 7,44 (tl) | $H_7$ |
| 7,49 (tl) et | $H_8$ |
| 7,63 (dl) | $H_6$ |
| 6,72 (s) | $C\underline{H}=C\underline{H}$ |
| 9,67 (m) | 2H mobiles |

**<u>EXEMPLE 5</u> : Ethanedioate de 5-[2-(diéthylamino) éthyl] 2,3-dihydro 3-(4-méthoxy phényl) 1,5-benzothiazépin-4(5H)-one.**

On opère comme à l'exemple 3 à partir de 2,86 g de produit obtenu comme au stade D de la préparation 1, 3,94 g de triphényl phosphine, 2,6 g d'azodicarboxylate de diéthyle et 1,76 g de diéthylaminoéthanol. On chromatographie l'extrait sec obtenu, (5 g) sur silice (éluant : chlorure de méthylène-méthanol : 95-5). On obtient 1,9 g de produit recherché sous forme de base.

Salification :

A une solution de 1,9 g de la base ci-dessus, dans 20 $cm^3$ d'isopropanol à chaud, on ajoute 623 mg d'acide oxalique en solution dans 5 $cm^3$ de méthanol. On évapore le méthanol, laisse en repos 3 heures, essore, lave avec 5 $cm^3$ d'isopropanol et 30 $cm^3$ d'éther éthylique. On obtient 2,1 g de produit brut F = 195 °C. On recristallise 2,5 g de produit obtenu comme ci-dessus, dans l'éthanol et obtient 2,2 g de produit recherché. F = 195 °C.

| Analyse pour $C_{24}H_{30}N_2O_6S$ = 474,608 | | | | | | | | |
|---|---|---|---|---|---|---|---|---|
| Calculés<br>Trouvés | C% | 60,74<br>60,6 | H% | 6,37<br>6,5 | N% | 5,90<br>5,9 | S% | 6,75<br>7,0 |

Spectre RMN (250 MHz, DMSO)

| | |
|---|---|
| 2-N-$CH_2$-C**H**$_3$ | 1,10 (t) |
| -$N^+$(**CH**$_2$)$_3$ | de 2,85 à 3,32 (m) 6H |
| ($C_6H_6$)-S-C**H**$_2$-C**H**- | de 3,35 à 3,77 (m) |
| O-C**H**$_3$ | 3,72 (s) |
| CO-N-C**H**$_2$ | 3,91 (m)<br>4,17 (m) |
| $H_2$' et $H_6$' | 6,84 (d,j = 8,5) |
| $H_3$' et $H_5$' | 7,24 (d,j = 8,5) |
| autres aromatiques | 7,37 (t) 1H<br>de 7,60 à 7,70 (m) 3H. |

**EXEMPLE 6 : (E)-butènedioate de 2,3-dihydro 5-[3-(diméthylamino) propyl] 3-(4-méthoxy phényl) 1,5-benzothiazépin-4(5H)-one.**

A une solution de 1,42 g de produit obtenu comme au stade D de la préparation 1, dans 7 $cm^3$ de diméthyl formamide, on ajoute 0,8 g d'hydrure de sodium en dispersion à 50 % dans l'huile (on mesure le volume d'hydrogène dégagé = 110 $cm^3$). On ajoute ensuite en refroidissant : 1,58 g de chlorhydrate de chlorure de 3-(diméthylamino) propane. On agite pendant 4 heures à 60 °C. On coule le mélange réactionnel dans 150 $cm^3$ d'eau, extrait par 3 fois 100 $cm^3$ d'acétate d'éthyle, lave à l'eau, sèche et évapore à sec. On obtient 2,3 g d'huile que l'on chromatographie sur silice (éluant : acétate d'éthyleméthanol-triéthylamine : 60-40-3). On obtient 988 mg de produit recherché sous forme de base.

Salification :

830 mg du produit obtenu ci-dessus, sont dissous dans 10 $cm^3$ d'isopropanol et ajoutés à une solution de 260 mg d'acide fumarique dans 10 $cm^3$ de méthanol bouillant. On agite puis laisse en repos 3 heures à température ambiante. On essore et obtient 860 mg de produit brut F = 182 °C, que l'on recristallise dans 35 $cm^3$ d'éthanol à reflux. On recueille 740 mg de produit recherché. F = 184 °C.

| Analyse pour $C_{25}H_{30}N_2O_6S$ = 486,583 | | | | | | | | |
|---|---|---|---|---|---|---|---|---|
| Calculés<br>Trouvés | C% | 61,71<br>61,7 | H% | 6,21<br>6,4 | N% | 5,76<br>5,7 | S% | 6,59<br>6,4 |

Spectre RMN (250 MHz, DMSO)

| | |
|---|---|
| 1,66 (m) | $CH_2$ central |
| 2,26 (s) | les $NH_3$ |
| 3,71 (s) | $-\overset{\|}{C}-O-C\underline{H}_3$ |
| 4,13 (m) | $CH_2-\underset{(C_6H_5)}{C\underline{H}}-CH_2$ |
| de 3,3 à 3,8 | les autres $CH_2$ |
| 6,54 (s) | les éthyléniques |
| 6,81 (d,j = 8,5 Hz) | aromatiques en ortho de $OCH_3$ |
| 7,22 (d,j = 8,5 Hz) | aromatiques en méta de $OCH_3$ |
| 7,33 (t) | $H_7$ ou $H_8$ |
| 7,67 (d) | $H_6$ ou $H_9$ |
| 7,59 (m) | 2H |

**EXEMPLE 7 : dichlorhydrate de 2,3-dihydro 3-(4-méthoxy phényl) 5-[2-(4-méthyl 1-piperazinyl) éthyl] 1,5-benzothiazépin-4(5H)-one.**

On opère comme à l'exemple 3 à partir de 2,86 g de produit obtenu au stade D de la préparation 1, en utilisant 3,94 g de triphényl phosphine, 2,6 g d'azodicarboxylate d'éthyle et 1,76 g de 1-[(2-hydroxy) éthyl] 4-méthyl pipérazine. Après chromatographie sur silice (éluant chlorure de méthylène-méthanol : 94-6), on obtient 1,32 g de produit attendu sous forme de base.

Salification :

A une solution de 1,3 g de la base obtenue ci-dessus, dans 10 $cm^3$ d'isopropanol, on ajoute 15 $cm^3$ d'une solution 3,6 N d'acide chlorhydrique dans l'acétate d'éthyle. On laisse au repos 4 heures à température ambiante. Après essorage, on obtient 1,10 g de produit recherché F = 205 °C, que l'on recristallise dans un mélange éther éthylique-méthanol (1-1). On obtient 800 mg de produit recherché F = 205 °C.

| Analyse pour $C_{23}H_{31}Cl_2N_3O_2S$ = 484,492 | | | | | | | | | | |
|---|---|---|---|---|---|---|---|---|---|---|
| Calculés<br>Trouvés | C% | 57,02<br>57,0 | H% | 6,45<br>6,6 | Cl% | 14,63<br>14,5 | N% | 8,67<br>8,5 | S% | 6,62<br>6,6 |

Spectre RMN (300 MHz, DMSO)

2,79 (s) N-C**H**$_3$

3,72 (s) O-C**H**$_3$

4,04 (m) }
4,27 (m) } CO-N-C**H**$_2$

6,84 (d) H$_2$' et H$_6$'

7,24 (d) H$_3$' et H$_5$'

7,38 (dt) }
7,61 (dt) } 4H aromatiques
7,68 (dt) }

11,86 (ep) < 2H mobiles

de 3,0 à 3,8 (m) les autres protons.

**EXEMPLE 8 : Ethanedioate de (±)-2,3-dihydro 3-(4-méthoxy phényl) 5-[2-[4-(2-méthoxy phényl) 1-pipérazinyl] éthyl] 1,5-benzothiazépin-4(5H)-one.**

On opère comme à l'exemple 3 à partir de 2,85 g de produit obtenu comme au stade D de la préparation 1, en utilisant 3,15 g de triphényl phosphine, 2,09 g d'azodicarboxylate de diéthyle et 2,84 g de 4-(2-méthoxy phényl) 1-pipérazine éthanol. On obtient, après chromatographie sur silice (éluant : cyclohexane-dioxanne 7-3) 1,23 g de produit attendu sous forme de base.

Salification :

On dissout 570 mg de produit sous forme de base, dans 30 cm$^3$ d'isopropanol et ajoute 143 mg d'acide oxalique en solution dans 1 cm$^3$ de méthanol. On concentre à 15 cm$^3$ au total. Après essorage, on obtient 419 mg de produit attendu (F = 128 °C) qui sont recristallisés dans 50 cm$^3$ d'éthanol. On concentre à 20 cm$^3$ au total. On obtient 260 mg de produit attendu F = 130 °C.

**EXEMPLE 9 : Chlorhydrate de (±)-5-[2-[[bis(1-méthyl éthyl)] amino] éthyl] 2,3-dihydro 3-(4-méthoxy phényl) 1,5-benzothiazépin-4(5H)-one.**

On opère comme à l'exemple 3 à partir de 2 g de produit obtenu au stade D de la préparation 1 en utilisant 2,2 g de triphényl phosphine, 1,22 g de diisopropyl amino éthanol et 1,46 g d'azodicarboxylate de diéthyle. On obtient, après chromatographie sur silice (éluant : chlorure de méthylène-méthanol : 95-5) 1,76 g de produit attendu sous forme de base.

Salification :

A une solution de 1,76 g de base dans 5 cm3 d'acétate d'éthyle, on ajoute une solution d'acide chlorhydrique dans l'acétate d'éthyle, jusqu'à pH 2. On obtient 1,28 g de produit brut F = 142 °C que l'on recristallise par deux fois dans un mélange éthanol-ether. On obtient 600 mg de produit attendu F = 160 °C.

| Analyse pour $C_{24}H_{33}ClN_2O_2S$ = 449,06 | | | | | | | | | | |
|---|---|---|---|---|---|---|---|---|---|---|
| Calculés<br>Trouvés | C% | 64,19<br>64,5 | H% | 7,41 7,6 | Cl% | 7,89<br>7,7 | N% | 6,24<br>6,1 | S% | 7,14<br>6,9 |

Produit solvaté à 3,8 %.

**EXEMPLE 10 : Chlorhydrate de (±)-2,3-dihydro 3-(3,4-diméthoxy phényl) 5-[2-(diméthylamino) éthyl] 1,5-benzothiazépin-4(5H)-one.**

On opère comme à l'exemple 3 à partir de 3,15 g de produit obtenu au stade D de la préparation 2 en utilisant 3,15 g de triphényl phosphine, 1,2 $cm^3$ de NN-diméthyl éthanol amine et 1,9 $cm^3$ d'azodicarboxylate de diéthyle. On obtient 2,1 g de produit attendu sous forme de base.

Salification :

A une solution de 2,1 g de la base obtenue ci-dessus, dans 9 $cm^3$ d'isopropanol, on ajoute de l'éthanol chlorhydrique jusqu'à pH 1. On obtient 1,2 g de produit recherché F 148 °C. On recristallise 1,2 g dans 8 $cm^3$ d'isopropanol et obtient 800 mg de produit F 150 °C.

Spectre RMN ($CDCl_3$, 250 MHz)

| | | |
|---|---|---|
| 3,47 (m) | 2H | $S-C\underline{H}_2-CH$ |
| 3,74 (dd) | 1H | $S-CH_2-C\underline{H}$ |
| 3,85 (s) et 3,87 (s) | | les $OC\underline{H}_3$ |
| 3,12 et 3,47 (m) | 2H | 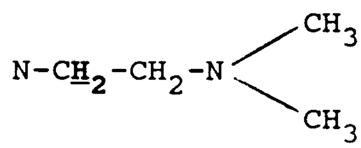 |
| 4,19 et 4,57 (m) | 2H | 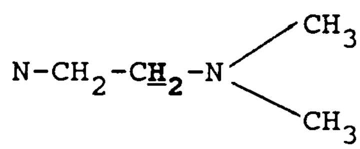 |
| 2,78 et 2,88 (s dédoublé) | | 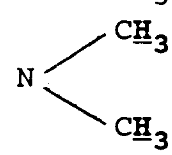 |
| 7,33-7,54-7,68 | 4H | les aromatiques |
| 6,83 (m) | 3H | autres aromatiques du phényl trisubstitué |
| 12,62 | | H mobile. |

Préparation 2 : 2,3-dihydro 3-(3,4-diméthoxy phényl) 1,5-benzothiazépin-4(5H)-one.

STADE A : 3,4-diméthoxy alpha-méthylène-benzèneacétate d'éthyle.

A une suspension de 0,1 mole d'éthylate de sodium, préparé à partir de 2,29 g de sodium dans 60 $cm^3$ de toluène séché sur siliporite, on ajoute : 28,7 g de diéthyl oxalate. On ajoute, ensuite, à 30 °/40 °C, 20 g de 3,4-diméthoxy phénylacétate d'éthyle. On maintient pendant 2 heures à reflux, refroidit ensuite à -30 °C et ajoute rapidement 24,6 $cm^3$ d'acide sulfurique 4N. On laisse revenir à -10 °C et ajoute quelques mg d'hydroquinone, 8 $cm^3$ d'aldéhyde formique, à 36 % en solution dans l'eau (stabilisé à 10 % de méthanol) et toujours à -10 °C lentement, une solution saturée de carbonate de potassium. On laisse revenir à température ambiante, agite 2 heures, ajoute 4 $cm^3$ d'aldéhyde formique à 36 % et agite 16 heures à température ambiante. On ajoute 180 $cm^3$ d'eau, décante, concentre à sec sous pression réduite. On obtient 21 g de produit attendu utilisé tel quel pour le stade suivant.

STADE B : Acide 3,4-diméthoxy alpha-méthylène benzèneacétique.

On opère comme au stade B de l'exemple 1, à partir de 18 g de produit obtenu au stade A. On obtient 8,8 g de produit recherché F = 128 °C, utilisé tel quel pour le stade suivant. On recristallise 540 mg de produit dans 15 $cm^3$ d'éther isopropylique. On obtient 300 mg de produit recherché F = 132 °C.

Spectre RMN ($CDCl_3$, 250 MHz)

3,90 (S) les $CH_3$-O
6,86 (d, j = 8) 1H
7,00 (d, j = 2) 1H
7,03 (dd, j = 8 et 2) 1H
5,99 (d, j = 1) -C-COOH
‖
6,49 (d, j = 1) C**H**$_2$
11,56 COO**H**

STADE C : Acide alpha-[(2-amino phényl thio) méthyl] 3,4-diméthoxy benzèneacétique.

On opère comme au stade C de l'exemple 1, à partir de 7,8 g du produit obtenu au stade B et obtient 9,1 g de produit recherché F = 162 °C, utilisé tel quel pour le stade suivant. On recristallise 1 g de produit dans 15 $cm^3$ d'acétonitrile et obtient 650 mg d'échantillon analytique F = 164 °C.

| Analyse pour $C_{17}H_{19}N_{O4}S$ = 333,33 | | | | | | | | |
|---|---|---|---|---|---|---|---|---|
| Calculés<br>Trouvés | C% | 61,24<br>61,1 | H% | 5,74<br>5,9 | N% | 4,20<br>4,4 | S% | 9,61<br>9,7 |

Spectre RMN (DMSO, 250 MHz)

S-C**H**$_2$-C 2,97 (dd, j = 6 et 13)
3,33 (dd, j = 9 et 13)
3,56 (dd, j = 6 et 9) 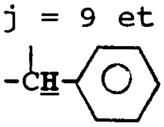
3,81 les C**H**$_3$-O
6,59 (t) (1H)
7,08 (t) (1H)
7,28 (d) (1H)
6,80 (m) (4H)
les aromatiques

STADE D : 2,3-dihydro 3-(3,4-diméthoxy phényl) 1,5-benzothiazépin-4(5H)-one.

Une solution de 9,1 g de produit obtenu au stade C, 180 $cm^3$ de chlorure de méthylène, 6,25 g de chlorhydrate de 1-éthyl 3-(3-diméthyl amino propyl) carbodiimide et 9 $cm^3$ de triéthyl amine, est agitée pendant 1 heure 30 minutes à température ambiante. On évapore le chlorure de méthylène et reprend le résidu avec 90 $cm^3$ d'acétate d'éthyle. On essore et obtient 7,8 g de produit attendu F = 174 °C. Un échantillon analytique a été obtenu par recristallisation de 200 mg de produit brut dans 10 $cm^3$ d'acétate d'éthyle. On obtient 120 mg de produit recherché F 184 °C.

| Analyse pour $C_{17}H_{17}NO_3S$ = 315,38 | | | | | | | | |
|---|---|---|---|---|---|---|---|---|
| Calculés<br>Trouvés | C% | 64,73<br>64,8 | H% | 5,43<br>5,4 | N% | 4,44<br>4,5 | S% | 10,16<br>10,3 |

7,16 (dd)
7,23 (td)
7,40 (td)
7,66 (dd) } les aromatiques
7,89 NH

**EXEMPLE 11 : Chlorhydrate de 3-(4-chloro phényl) 2,3-dihydro 5-[2-(diméthylamino) éthyl] 1,5-benzothiazépin-4(5H)-one.**

On opère comme à l'exemple 3 à partir de 3,5 g du produit obtenu au stade D de la préparation 3 en utilisant 1,8 $cm^3$ de N-diméthyl éthanol amine, 4,72 g de triphényl phosphine et 2,83 $cm^3$ de diéthyl azodicarboxylate. On obtient 3 g de produit attendu F = 148 ° C, sous forme de base.

Salification :

On place en suspension dans 30 $cm^3$ d'isopropanol les 3 g de la base obtenue ci-dessus et ajoute jusqu'à pH 1, une solution d'acide chlorhydrique dans l'éthanol. On essore, lave à l'isopropanol et obtient 2,7 g de produit recherché. F 248 ° C.

| Analyse pour $C_{19}H_{21}ClN_2SO$, HCl = 397,36 | | | | | | | | | | |
|---|---|---|---|---|---|---|---|---|---|---|
| Calculés<br>Trouvés | C% | 57,42<br>57,4 | H% | 5,58<br>5,6 | Cl% | 17,84<br>17,9 | N% | 7,05<br>7,0 | S% | 8,07<br>7,8 |

Spectre RMN (DMSO, 250 MHz)

| | | | | |
|---|---|---|---|---|
| les 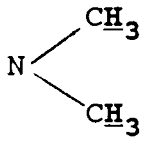 | | 2,75 | | |
| 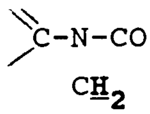 | | ≃ 4,01 (m) | | (1H) |
| | et | 4,27 (m) | | (1H) |
| $S-C\underline{H}_2-CH$ | | ≃ 3,06 (m) | | (1H) |
| et | | 3,34 (m) | | (1H) |
| $>N-C\underline{H}_2-CH_2$ | | 3,30 (m) | | (2H) |
| 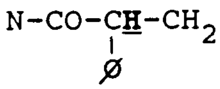 | | 3,81 | | |
| Aromatiques | de | 7,30 à 7,50 | | (5H) |
| | de | 7,55 à 7,8 | | (3H) |

Spectre IR ($CHCl_3$ sur Nicolet)

Absence NH type lactame. Présence de $NH^+$

C=O 1669 $cm^{-1}$

Aromatiques 1594 $cm^{-1}$
1581 $cm^{-1}$
1568 $cm^{-1}$
1494 $cm^{-1}$

Préparation 3 : 2,3-dihydro 3-(4-chloro phényl) 1,5-benzothiazépin-4(5H)-one.

STADE A : 4-chloro alpha-méthylène benzèneacétate de méthyle.

A une suspension de 0,351 mole de méthylate de sodium (préparé à partir de 8,06 g de sodium) dans 510 $cm^3$ d'éther, on ajoute une solution de 51 g de 4-chlorophényl acétate de méthyle, 44 g de diméthyl oxalate et 320 $cm^3$ d'éther. On agite 17 heures à reflux, ajoute 250 $cm^3$ d'éther. On refroidit et ajoute lentement 250 $cm^3$ d'acide chlorhydrique 2N jusqu'à pH 1. On décante, lave avec 50 $cm^3$ d'acide chlorhydrique 2N puis à l'eau saturée de chlorure de sodium, on évapore à sec et obtient 69 g de produit huileux que l'on met en suspension dans 500 $cm^3$ d'eau (en présence de traces d'hydroquinone), ajoute 50 $cm^3$ d'aldéhyde formique à 40 % et agite 3 heures à température ambiante. On extrait à l'acétate d'éthyle, lave à l'eau, sèche et évapore à sec. On obtient 55 g de résidu que l'on chromatographie sur silice (éluant cyclohexane-acétate d'éthyle : 95-5). On obtient 33 g de produit recherché que l'on utilise tel quel pour le stade suivant.

STADE B : Acide 4-chloro alpha-méthylène benzèneacétique.

A une solution de 700 $cm^3$ de tétrahydrofuranne et 15 g du produit obtenu au stade A, on ajoute 530 $cm^3$ d'une solution 0,33 M de potasse. On agite pendant 76 heures à température ambiante. On évapore le tétrahydrofuranne et acidifie avec de l'acide chlorhydrique 2N. On essore, lave à l'eau, sèche et obtient 11,75 g de produit attendu F = 110 °C, que l'on utilise tel quel pour le stade suivant.

Un échantillon analytique est préparé par recristallisation de 350 mg de produit dans 50 $cm^3$ d'eau. On obtient 200 mg de produit. F = 110 °C.

| Analyse pour $C_9H_7ClO_2$ = 182,6 | | | | | | |
|---|---|---|---|---|---|---|
| Calculés<br>Trouvés | C% | 59,19<br>59,1 | H% | 3,86<br>3,8 | N% | 19,41<br>19,5 |

STADE C : Acide alpha-[[(2-amino phényl) thio] méthyl] 4-chlorobenzèneacétique.

On agite 17 heures à reflux une solution de 6 g de l'acide obtenu au stade B, dans 90 $cm^3$ d'éthanol avec 3,5 $cm^3$ de 2-aminothiophénol. On évapore à sec et reprend par 50 $cm^3$ de chlorure de méthylène à 5 % de méthanol. On essore et obtient 6 g de produit recherché F = 142 °C, utilisé tel quel pour le stade suivant.

On a préparé un échantillon analytique par recristallisation de 350 mg de produit dans 20 $cm^3$ de cyclohexane et 2 $cm^3$ d'isopropanol. On recueille 220 mg de produit. F = 142 °C.

| Analyse pour $C_{15}H_{14}ClNO_2S$ = 307,789 | | | | | | | | | | |
|---|---|---|---|---|---|---|---|---|---|---|
| Calculés<br>Trouvés | C% | 58,53<br>58,5 | H% | 4,58<br>4,5 | Cl% | 11,52<br>11,7 | N% | 4,55<br>4,5 | S% | 10,41<br>10,2 |

STADE D : 2,3-dihydro 3-(4-chloro phényl) 1,5-benzothiazépin-4(5H)-one.

On agite 2 heures 30 à température ambiante, une solution de 5 g d'acide obtenu au stade C, 125 $cm^3$ de chlorure de méthylène et 3,73 g de chlorhydrate de 1-éthyl 3-(3-diméthyl amino propyl) carbodiimide. On évapore à sec et reprend le résidu avec 25 $cm^3$ d'éthanol. On essore et lave à l'éthanol. On obtient 3 g de produit recherché F = 180 °C. Par chromatographie du filtrat sur silice (éluant : acétate d'éthyle), on recueille un second jet de 500 mg F = 180 °C, après cristallisation dans 10 $cm^3$ d'éthanol. Le produit est utilisé tel quel pour le stade suivant. Un échantillon analytique a été préparé par recristallisation de 350 mg de produit dans 10 $cm^3$ d'éthanol. On recueille 230 mg de produit fondant à 180 °C.

| Analyse pour $C_{15}H_{12}ClNSO$ = 289,77 | | | | | | | | | | |
|---|---|---|---|---|---|---|---|---|---|---|
| Calculés<br>Trouvés | C% | 62,17<br>62,3 | H% | 4,17<br>4,2 | N% | 4,83<br>4,9 | Cl% | 12,23<br>12,3 | S% | 11,06<br>10,74 |

**EXEMPLE 12 : Chlorhydrate de 2,3-dihydro 5-[2-(diméthylamino) éthyl] 3-(2-méthoxy phényl) 1,5-benzothiazépin-4(5H)-one.**

On opère comme au stade 3 à partir de 2,85 g du produit obtenu au stade E de la préparation 4, en utilisant 1,5 $cm^3$ de N-diméthyl éthanol amine, 3,93 g de triphényl phosphine et 2,36 $cm^3$ d'azodicarboxylate de diéthyle. On obtient après chromatographie sur silice (éluant : chlorure de méthylène-méthanol : 90-10) 1,6 g de produit recherché sous forme de base.

Salification :

900 mg de la base obtenue ci-dessus sont dissous dans 5 $cm^3$ d'isopropanol. On ajoute de l'éthanol chlorhydrique jusqu'à pH 1. On essore, lave à l'isopropanol et sèche. On obtient 800 mg de produit recherché F ≃ 195 °C. Après une recristallisation de ces 800 mg dans 5 $cm^3$ d'isopropanol, on recueille 630 mg F = 195 °C.

| Analyse pour $C_{20}H_{24}N_2O_2S$, HCl = 392,94 | | | | | | | | | | |
|---|---|---|---|---|---|---|---|---|---|---|
| Calculés<br>Trouvés | C% | 61,13<br>60,8 | H% | 6,41<br>6,5 | N% | 7,13<br>7,0 | Cl% | 9,02<br>9,3 | S% | 8,16<br>7,9 |

Perte sous vide à 100 °C 2,6 %.

<u>Spectre RMN</u> ($CDCl_3$, 250 MHz)

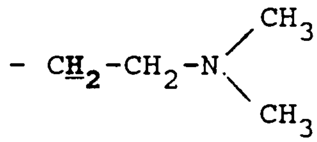 4,20 (m) - 4,59 (m)

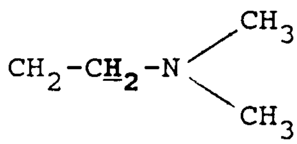 3,12 (m) - 3,41 (m)

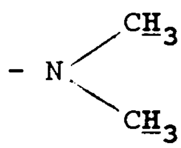 2,81

O-C**H**$_3$ 3,70

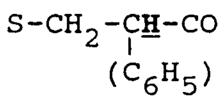 4,20 (t, j = 10 Hz)

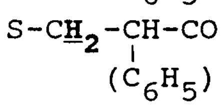 3,53 (d)

1H en ortho de $OCH_3$ 6,80 (d) 1H

Les aromatiques
- 6,97 (t) 1H
- 7,2 à 7,35 3H
- 7,53 (m) 2H
- 7,67 (d,d) 1H

<u>Préparation 4</u> : 2,3-dihydro 3-(2-méthoxy phényl) 1,5-benzothiazépin-4(5H)-one.

<u>STADE A</u> : 2-méthoxy phényl acétate de méthyle.

On agite pendant 52 heures, à températue ambiante, une solution de 40 g d'acide 2-méthoxy phényl acétique, 160 cm$^3$ de méthanol et 8 cm$^3$ d'une solution aqueuse d'acide sulfurique (à 5 % en volume). On concentre ensuite à ≃ 100 cm$^3$ au total puis ajoute 400 cm$^3$ d'eau. On extrait avec du chlorure de méthylène et lave la phase organique avec une solution normale de carbonate de potassium et à l'eau. On évapore à sec sous pression réduite et obtient 41 g de produit utilisé tel quel pour le stade suivant.

<u>STADE B</u> : 2-méthoxy alpha-méthylène benzèneacétate de méthyle.

A une suspension de 0,280 mole de méthylate de sodium (préparé à partir de 6,4 g de sodium) dans 400 cm$^3$ d'éther, on ajoute une solution de 40 g du produit obtenu au stade A, 35 g de diméthyl oxalate dans 250 cm$^3$ d'éther. On agite 17 heures à reflux, refroidit, ajoute 250 cm$^3$ d'éther puis 200 cm$^3$ d'acide chlorhydrique 2N jusqu'à pH 1. On décante, lave à l'acide chlorhydrique 2N, puis avec une solution saturée

de chlorure de sodium. On évapore à sec et obtient 51 g d'huile que l'on met en suspension dans 400 $cm^3$ d'eau en présence de traces d'hydroquinone et ajoute : 40 $cm^3$ d'aldéhyde formique à 40 % et 33,4 g de carbonate de potassium. On agite pendant 3 heures à température ambiante. On extrait avec de l'acétate d'éthyle, lave la phase organique à l'eau, sèche et évapore. Le résidu est chromatographié sur silice (éluant : cyclohexane-acétate d'éthyle 95-5). On recueille 24 g de produit recherché utilisé tel quel pour le stade suivant.

STADE C : Acide 2-méthoxy alpha-méthylène benzéneacétique.

A une solution de 21 g du produit obtenu au stade B, dans 1 litre de tétrahydrofuranne, on ajoute 765 $cm^3$ d'une solution aqueuse (0,33 M) de potasse. On agite 48 heures à température ambiante. On évapore le tétrahydrofuranne, refroidit la phase aqueuse et acidifie avec de l'acide chlorhydrique 2N. On essore, lave à l'eau et sèche. On obtient 16,8 g de produit utilisé tel quel pour le stade suivant.

Un échantillon analytique a été préparé par recristallisation par chaud et froid de 1,6 g de produit dans 30 $cm^3$ d'éther isopropylique. On obtient 0,8 g de produit F = 146 °C.

| Analyse pour $C_{10}H_{10}O_3$ = 178,18 | | | | |
|---|---|---|---|---|
| Calculés<br>Trouvés | C% | 67,40<br>67,4 | H% | 5,65<br>5,8 |

STADE D : Acide alpha-[[(2-amino phényl) thio] méthyl] 2-méthoxy benzèneacétique.

On chauffe 4 heures, à reflux une solution de 230 $cm^3$ d'éthanol, 16,3 g du produit obtenu au stade C et 9,8 cm3 de 2-aminothio phénol. On évapore l'éthanol et chromatographie le résidu (28 g) sur silice (éluant : cyclohexane-acétate d'éthyle (6-4)). On obtient 14 g de produit que l'on cristallise dans 25 $cm^3$ de cyclohexane et 1 $cm_3$ d'isopropanol. On obtient 12,5 g de produit recherché F = 85 °C, que l'on utilise tel quel pour le stade suivant.

Un échantillon analytique a été préparé par recristallisation de 180 mg du produit obtenu ci-dessus, dans 3 $cm^3$ de cyclohexane et 0,1 $cm^3$ d'isopropanol. On recueille 80 mg de produit purifié F = 90 °C.

| Analyse pour $C_{16}H_{17}NO_3S$ = 303,372 | | | | | | | | |
|---|---|---|---|---|---|---|---|---|
| Calculés<br>Trouvés | C% | 63,34<br>63,5 | H% | 5,65<br>5,8 | N% | 4,61<br>4,6 | S% | 10,57<br>10,5 |

Spectre RMN ($CDCl_3$, 60 MHz)

| | |
|---|---|
| 169 à 213,5 | S-C**H**$_2$-CH |
| 241 à 256 | S-$CH_2$-C**H** |
| 220,5 | O-C**H**$_3$ |
| 309 | H du COO**H**<br>H du N**H**$_2$ |
| 388 à 444 | les aromatiques. |

STADE E : 2,3-dihydro 3-(2-méthoxy phényl) 1,5-benzothiazépin-4(5H)-one.

On agite pendant 6 heures à température ambiante, 11,7 g d'acide obtenu au stade D, 7,95 g de dicyclohexylcarbodiimide et 350 $cm^3$ d'éthanol. On évapore à sec et reprend le résidu par 1500 $cm^3$ de chlorure de méthylène, filtre l'insoluble, concentre le filtrat à sec, reprend le résidu avec 1500 $cm^3$ d'éthanol et essore. On recueille 9,6 g de produit recherché F = 219 °C que l'on utilise tel quel pour le stade suivant.

On a préparé un échantillon analytique en recristallisant 350 mg de produit dans 40 $cm^3$ d'éthanol. On obtient 240 mg de la structure recherchée F = 223 °C.

| Analyse pour $C_{16}H_{15}NO_2S$ = 285,35 | | | | | | | | |
|---|---|---|---|---|---|---|---|---|
| Calculés<br>Trouvés | C% | 67,34<br>67,2 | H% | 5,30<br>5,2 | N% | 4,91<br>4,9 | S% | 11,23<br>11,3 |

<u>Spectre RMN</u> (DMSO, 250 MHz)

| | |
|---|---|
| S-C**H**$_2$-CH-CO<br>(C$_6$H$_5$) | 3,50 à 3,72 |
| S-CH$_2$-C**H**-CO<br>(C$_6$H$_5$) | 4,13 |
| O-C**H**$_3$ | 3,63 |
| Aromatiques | 6,84 à 7,61. |

**<u>EXEMPLE 13</u> : (E)-2-butènedioate de 2,3-dihydro 5-[2-(diméthylamino) éthyl] 3-(4-méthoxy phényl) 1,5-benzothiazépin-4(5H)-one.**

On opère comme à l'exemple 3, à partir de 3 g du produit obtenu comme au stade D de la préparation 1, en utilisant : 4,11 g de triphényl phosphine, 1,58 $cm^3$ de N-diméthyl éthanol amine et 2,47 g d'azodicarboxylate de diéthyle. Après chromatographie sur silice (éluant : chlorure de méthylène-méthanol 95-5) on obtient 2,8 g de produit recherché sous forme de base.

<u>Salification</u> :

On dissout 2,8 g du produit obtenu ci-dessus, dans 30 $cm^3$ d'acétate d'éthyle et ajoute une solution de 0,91 g d'acide fumarique dans 25 $cm^3$ d'éthanol. Après essorage on obtient 1,8 g de produit recherché. F = 162 °C. On recristallise le produit ci-dessus dans 225 $cm^3$ d'acétate d'éthyle, concentre à 100 $cm^3$. On obtient 1,5 g. F = 162 °C.

| Analyse pour $C_{24}H_{28}N_2O_6S$ = 472,54 | | | | | | | | |
|---|---|---|---|---|---|---|---|---|
| Calculés<br>Trouvés | C% | 60,99<br>61,0 | H% | 5,97<br>6,0 | N% | 5,92<br>5,9 | S% | 6,78<br>6,6 |

<u>Spectre RMN</u> (DMSO, 250 MHz)

| | | | |
|---|---|---|---|
| 2,43 à 4,16 | | N-C**H**$_2$-C**H**$_2$-N< | |
| 2,27 | (6H) | 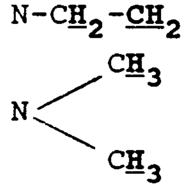 | |
| 3,71 | (3H) | OC**H**$_3$ | Les aromatiques en alpha et béta de $OCH_3$ |
| 6,82 | (2H) | (d, j = 8,5) | |
| 7,22 | (2H) | (d, j = 8,5) | |

| | |
|---|---|
| 2,43 à 4,16 | S-C**H**$_2$-C**H**- |
| 7,33 (t)<br>7,53 (t)<br>≃ 7,64 (d) | les autres aromatiques |

**EXEMPLE 14 : (E)-2-butènedioate de (±)-2,3-dihydro 5-[2-(diméthylamino) éthyl] 3-(4-méthoxy phényl) 1,5-benzothiazépin-4(5H)-one.**

STADE A : (±)-2,3-dihydro 3-(4-méthoxy phényl) 4-oxo-1,5-benzothiazépin-5(4H)-acétate d'éthyle.

A une solution (6,84 g) de produit obtenu comme au stade D de la préparation 1, dans 30 $cm^3$ de diméthyl formamide, on ajoute, entre +15 et +20°C, 1,35 g d'hydrure de sodium en dispersion à 50 % dans l'huile. On agite 15 minutes à 20°C (volume d'hydrogène dégagé : 540 $cm^3$). On refroidit à +5°C/+10°C et ajoute 3,12 $cm^3$ de bromacétate d'éthyle. On agite 1 heure à température ambiante, verse sur 200 $cm^3$ d'eau extrait à l'éther, lave à l'eau et obtient, après évaporation sous pression réduite, 9 g d'huile que l'on chromatographie sur silice (éluant : chlorure de méthylène-méthanol : 99-1). On obtient 6,8 g de produit attendu utilisé tel quel pour le stade B.

STADE B : (±)-acide 2,3-dihydro 3-(4-méthoxy phényl) 4-oxo-1,5-benzothiazépin-5(4H)-acétique.

On dissout 6,8 g du produit obtenu au stade A, dans 136 $cm^3$ d'un mélange à parties égales d'eau, d'acide acétique et d'acide sulfurique. On agite pendant 19 heures à température ambiante. On ajoute lentement 400 $cm^3$ d'eau, agite 10 minutes, et essore. On obtient 5,3 g de produit attendu F = 180°C utilisé tel quel pour le stade suivant (stade C).

Un échantillon analytique a été préparé par deux recristallisation sucessives dans 20 vol. d'éther à reflux à partir de 2,7 g d'acide obtenu ci-dessus. On recueille 1,15 g de produit F = 190°C (peu net).

| Analyse pour $C_{18}H_{17}NO_4S$ = 343,40 | | | | | | | | |
|---|---|---|---|---|---|---|---|---|
| Calculés<br>Trouvés | C% | 62,96<br>62,9 | H% | 4,99<br>5,0 | N% | 4,08<br>3,9 | S% | 9,33<br>9,3 |

STADE C : 2,3-dihydro 5-(2-hydroxy éthyl) 3-(4-méthoxy phényl) 1,5-benzothiazépin-4(5H)-one.

A une solution de 3,44 g d'acide obtenu au stade B, dans 34,4 $cm^3$ de tétrahydrofuranne, on ajoute à +10°C, en 10 minutes 7,5 $cm^3$ de complexe borane-diméthyl sulfure 2M dans le tétrahydrofuranne. Après 18 heures d'agitation à température ambiante, on ajoute de nouveau 1 $cm^3$ de la solution de complexe borane-diméthylsulfure et agite encore 6 heures à température ambiante. On ajoute 5 $cm^3$ de méthanol, évapore à sec sous pression réduite, ajoute 100 $cm^3$ d'eau et extrait par trois fois 40 $cm^3$ de chlorure de méthylène. On filtre, sèche et évapore à sec sous pression réduite. On cristallise le résidu (3,8 g) dans 5 $cm^3$ d'éther et obtient : 1,9 g de produit attendu F = 133°C, utilisé tel quel pour l'étape suivante.

Un échantillon analytique est préparé par recristallisation de 150 mg de produit ci-dessus, dans l'éther. On obtient 125 mg de produit recherché. F = 135°C.

| Analyse pour $C_{18}H_{19}NO_3S$ = 329,40 | | | | | | | | |
|---|---|---|---|---|---|---|---|---|
| Calculés<br>Trouvés | C% | 65,63<br>65,5 | H% | 5,81<br>5,8 | N% | 4,25<br>4,1 | S% | 9,73<br>9,7 |

STADE D : 2,3-dihydro 5-(2-chloro éthyl) 3-(4-méthoxy phényl) 1,5-benzothiazépin-4(5H)-one.

A une solution de 329 mg de produit obtenu au stade C, dans 3,3 $cm^3$ de chlorure de méthylène, on ajoute 0,14 $cm^3$ de chlorure de thionyle. On agite 15 minutes à température ambiante puis évapore à sec sous pression réduite. On reprend le résidu avec 25 $cm^3$ d'éther, chauffe à reflux, traite avec du charbon actif, filtre et concentre à 7 $cm^3$. On essore et obtient 300 mg de produit attendu F ≃ 70 °C, utilisé tel quel pour le stade E.

Un échantillon analytique a été préparé par dissolution de 300 mg du produit ci-dessus dans 10 $cm^3$ de méthyl éthyl cétone à chaud, préparé en concentration à 2 $cm^3$ puis addition de 2 $cm^3$ de pentane. On recueille 65 mg de produit recherché F ≃ 70 °C après essorage.

| Analyse pour $C_{18}H_{18}NO_2ClS$ = 347,85 + 1/2 mole de méthyl éthyl cétone | | | | | | | | |
|---|---|---|---|---|---|---|---|---|
| Calculés<br>Trouvés | C% | 62,57<br>62,5 | H% | 5,78<br>5,8 | N% | 3,65<br>3,7 | Cl% | 9,23<br>9,3 |

STADE E : (E)-2-butène dioate de (±)-2,3-dihydro 5-[2-(diméthylamino) éthyl] 3-(4-méthoxy phényl) 1,5-benzothiazépin-4(5H)-one.

Une suspension de 348 mg du produit obtenu au stade D avec 1,7 $cm^3$ de diméthylamine à 33 % dans l'éthanol est agitée pendant 30 minutes à température ambiante en appareillage clos et 18 heures à 50 °C. On évapore à sec sous pression réduite, ajoute 40 $cm^3$ d'une solution saturée de bicarbonate de sodium, extrait au chlorure de méthylène, lave à l'eau, sèche et évapore à sec sous pression réduite. On chromatographie le résidu (280 mg) sur silice (éluant : chlorure de méthylène-méthanol 95-5) et recueille 43 mg de produit attendu sous forme de base.

Salification :

A une solution de 43 mg de la base obtenue ci-dessus dans 3 $cm^3$ de méthanol, on ajoute 14 mg d'acide fumarique et 15 $cm^3$ d'acétate d'éthyle. On concentre à 4 $cm^3$, essore, lave à l'acétate d'éthyle puis à l'éther. On obtient 40 mg de produit recherché. F = 162 °C.

**EXEMPLE 15 : Chlorhydrate de (±)-2,3-dihydro 5-[2-(diméthylamino) éthyl] 3-(4-hydroxy phényl) 1,5-benzothiazépin-4(5H)-one.**

On traite 2,1 g du produit obtenu à l'exemple 13 avec 20 $cm^3$ d'acide bromhydrique concentré à température ambiante pendant 24 heures, verse le milieu réactionnel sur 100 g d'eau et glace puis neutralise le milieu par addition de bicarbonate de sodium. On extrait avec du chlorure de méthylène, lave avec 2 fois 20 $cm^3$ d'eau, sèche et évapore à sec sous pression réduite. On obtient 1,54 g de produit attendu sous forme de base.

Salification :

A une solution de 1,5 g du produit obtenu ci-dessus, dans 10 $cm^3$ de méthyl éthyl cétone, on ajoute une solution d'acide chlorhydrique dans l'acétate d'éthyle jusqu'à pH1. On évapore l'acétate d'éthyle en maintenant le volume constant par addition de méthyl éthyl cétone. On essore, lave à la méthyl éthyl cétone puis l'éther éthylique. On obtient 740 mg de produit recherché. F ≃ 220 °C.

Un échantillon analytique a été préparé à partir de 1,22 g de produit obtenu comme ci-dessus, que l'on recristallise dans 40 $cm^3$ d'isopropanol. On recueille 900 mg de produit pur. F = 240 °C.

| Analyse pour $C_{19}H_{23}ClN_2O_2S$ = 378,924 | | | | | | | | | | |
|---|---|---|---|---|---|---|---|---|---|---|
| Calculés<br>Trouvés | C% | 60,23<br>60,1 | H% | 6,12<br>6,2 | N% | 9,36<br>9,4 | Cl% | 7,39<br>7,3 | S% | 8,46<br>8,4 |

Spectre RMN (DMSO, 250 MHz)

| | |
|---|---|
| 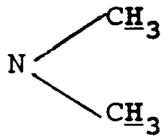 | 2,75 (s) |
| les 3 $CH_2$ et N | 3,03 (m) 1H |
| -C$\underline{H}$-C=O | 3,30 à 3,50 (m) 3H |
| ($C_6H_5$) | 3,62 (m) 1H |
| | 3,99 (m) 1H |
| | 4,27 (m) 1H |
| 2H mobiles | 9,41 (s) 1H |
| | 10,68 (sl) 1H |
| les aromatiques | de 7,09 à 7,67 |

**EXEMPLE 16 : Chlorhydrate de (±) N-(2-(diéthylamino éthyl) 2,3-dihydro 3-(4-méthoxyphénhyl) 4-oxo 1,5-benzothiazépin-5(4H)-acétamide.**

On agite pendant 1 heure et demie 1,71 g d'acide (±) 2,3-dihydro 3-(4-méthoxyphényl) 4-oxo 1,5-benzothazépin-5-(4H)-acétique préparé comme au stade B de l'exemple 14, 17 $cm^3$ de chlorure de méthylène, 1,74 g de N,N-diéthyléthylène diamine et 3,82 g de chlorhydrate de 1-éthyl 3-(diméthylaminopropyl) carbodiimide. On verse la solution sur 50 $cm^3$ d'eau, ajoute 10 $cm^3$ d'une solution aqueuse saturée de bicarbonate de sodium, extrait au chlorure de méthylène, lave à l'eau, sèche et élimine le solvant sous pression réduite. On obtient 3,6 g de produit brut que l'on chromatographie sur silice (éluant : chlorure de méthylène-méthanol 8-2). On obtient 1,6 g de produit attendu sous forme de base. On dissout la base dans 5 $cm^3$ d'isopropanol, ajoute une solution d'acide chlorhydrique dans l'acétate d'éthyle. On recueille 1,5 g de chlorhydrate attendu que l'on recristallise dans l'isopropanol et obtient 525 mg de produit pur. F 160 ° C.

| Analyse pour $C_{24}H_{31}N_3O_3S$, HCl | | | | | | | | | | |
|---|---|---|---|---|---|---|---|---|---|---|
| Calculés<br>Trouvés | C% | 60,30<br>60,0 | H% | 6,75<br>6,7 | Cl% | 7,42<br>7,2 | N% | 8,79<br>8,8 | S% | 6,71<br>6,7 |

| | |
|---|---|
| 1,19 (t) | $\underline{CH}_3-CH_2$ |
| 1,28 (t) | |
| 3,76 (s) | $OCH_3$ |
| 4,25 (d,J=17 Hz) | $N-CH_2-C(=O)-$ |
| 4,58 (d,J=17 Hz) | |
| 6,82 (d) | 2H en ortho de $OCH_3$ |
| 7,31 (d) | 2H en méta de $OCH_3$ |
| 3,4 à 4,0 (m) | 5H autres $CH_2$ et $\gamma$-$\underline{CH}-C(=O)$ |
| 2,8 à 3,2 (m) | 6H |
| 7,51 (m) | 2H |
| 7,68 (d) | 1H protons aromatiques |
| 7,31 (m) | 1H |
| 8,67 (t. mobile) | $N-\underline{H}-CH_2$ |
| 11,09 (s,l) | proton mobile |

**EXEMPLE 17 : (E) 2-butènedioate de (±) 2,3-dihydro 5-[(4-diméthylamino) butyl] 3-(4-méthoxyphényl) 1,5-benzothiazépin-4(5H)-one.**

Dans une solution comprenant 13,8 g de triphénylphosphine dans 350 $cm^3$ de tétrahydrofuranne, on ajoute à température ambiante en 15 minutes, 9 g de diéthylazodicarboxylate en solution dans 150 $cm^3$ de tétrahydrofuranne. On agite 10 minutes puis ajoute en 3 minutes 10 g de 2,3-dihydro 3-(4-méthoxyphényl) 1,5-benzothiazépin-4(5H)-one obtenue comme au stade D de la préparation 1, agite 10 minutes puis ajoute 6 g de diméthylaminobutanol. Après 5 heures d'agitation à température ambiante, on évapore le tétrahydrofuranne, extrait à l'aide d'acide chlorhydrique 2N, lave à l'acétate d'éthyle, réunit les phases aqueuses, alcalinise à l'aide de lessive de soude, extrait au chlorure de méthylène, lave avec une solution aqueuse de chlorure de sodium, sèche et évapore à sec. On obtient 5,7 g de produit brut que l'on chromatographie sur silice (éluant : chlorure de méthylène-méthanol 90-10). On obtient 2 g de produit attendu sous forme de base que l'on reprend dans l'isopropanol et ajoute 0,6 g d'acide fumarique. On obtient 1,35 g de produit attendu F = 144 °C après recristallisation dans l'isopropanol ou dans l'acétonitrile.

| Analyse : $C_{26}H_{32}O_6N_2S$ | | | | | | | | |
|---|---|---|---|---|---|---|---|---|
| Calculés<br>Trouvés | C% | 62,38<br>62,4 | H% | 6,44<br>6,2 | N% | 5,59<br>5,7 | S% | 6,40<br>6,3 |

**EXEMPLE 18 : Ethanedioate de (±) 2,3-dihydro 5-[2-[[2-(3,4-diméthoxyphényl) éthyl] méthylamino] éthyl] 3-(4-méthoxyphényl) 1,5-benzothiazépin-4(5H)-one.**

On agite 5 minutes à 140 °C 800 mg de 2,3-dihydro 5-(2-chloroéthyl) 3-(4-méthoxyphényl) 1,5-benzothiazépin-4-(5H)-one préparé comme au stade D de l'exemple 14 et 1,6 g de 3,4-diméthoxy béta-phénéthylméthylamine. On refroidit la solution obtenue, reprend dans le chlorure de méthylène, lave avec une solution aqueuse saturée de bicarbonate de soude, sèche et évapore à sec sous pression réduite. On recueille 2,25 g de produit brut que l'on chromatographie sur silice (éluant : chlorure de méthylène-méthanol 9-1) et obtient 900 mg de produit attendu sous forme de base que l'on dissout dans 9 $cm^3$ de méthanol, ajoute 223 mg d'acide oxalique, chauffe, ajoute 40 $cm^3$ d'isopropanol, concentre à 20 $cm^3$ et amorce la cristallisation. On abandonne 4 heures à température ambiante, essore et recueille 550 mg de produit attendu. F = 180-190 °C après recristallisation dans le méthanol et l'isopropanol puis le méthanol et l'éther.

| Analyse : $C_{31}H_{36}N_2O_8S$ | | | | | | | | |
|---|---|---|---|---|---|---|---|---|
| Calculés<br>Trouvés | C% | 62,40<br>62,2 | H% | 6,08<br>6,1 | N% | 4,70<br>4,6 | S% | 5,37<br>5,4 |

**EXEMPLE 19 : Ethanedioate de (±) 2,3-dihydro 5-[2-(diméthylamino) éthyl] 3-(3-méthoxyphényl) 1,5-benzothiazépin-4(5H)-one.**

On ajoute en 10 minutes à température ambiante 1,04 g de diéthylazadicarboxylate en solution dans 3 cm$^3$ de tétrahydrofuranne, dans une suspension comprenant 1,43 g de 2,3-dihydro 3-(3-méthoxyphényl) 1,5-benzothiazépin-4(5H)-one, 25 cm$^3$ de tétrahydrofuranne, 535 mg de diméthylamino éthanol et 1,57 g de triphénylphosphine. On laisse revenir à température ambiante, agite 24 heures, évapore le tétrahydrofuranne, ajoute 30 cm$^3$ d'acide chlorhydrique 2N, extrait à l'acétate d'éthyle, lave de nouveau à l'acide chlorhydrique, réunit les phases aqueuses, refroidit à 4 °C, alcalinise à l'aide de lessive de soude, extrait à l'acétate d'éthyle, lave à l'eau, sèche et évapore à sec sous pression réduite. On obtient 1,6 g de produit brut que l'on chromatographie sur silice (éluant : cyclohexane-dioxanne 60-40). On recueille 1,05 g de produit attendu sous forme de base (rf = 0,10) que l'on dissout dans 20 cm$^3$ d'isopropanol. On ajoute 371 mg d'acide oxalique en solution dans le méthanol. On concentre partiellement, amorce la cristallisation et obtient après essorage 1,2 g de produit attendu. F = 208 °C après recristallisation dans l'éthanol.

| Analyse : $C_{20}H_{24}N_2O_2S$, $C_2H_2O_4$ (produit séché à 150 °C) | | | | | | | | |
|---|---|---|---|---|---|---|---|---|
| Calculés<br>Trouvés | C% | 59,18<br>59,3 | H% | 5,87<br>5,9 | N% | 6,27<br>6,3 | S% | 7,18<br>7,0 |

**La 2,3-dihydro 3-(3-méthoxyphényl) 1,5-benzothiazépin-4(5H)-one utilisée au départ de l'exemple 19 a été préparée comme suit :**

**STADE A** : (3-méthoxyphényl) propanedioate de diméthyle.

On agite 1,48 g de sodium dans 50 cm$^3$ de méthanol jusqu'à obtention d'une solution. On concentre à sec, ajoute 40 cm$^3$ de toluène, évapore à sec et ajoute 100 cm$^3$ d'éther éthylique. On ajoute ensuite en 15 minutes à température ambiante, une solution comprenant 9 g de 3-méthoxypohénylacétate et 8 g de diméthyloxalate dans 65 cm$^3$ d'éther éthylique. On agite pendant 21 heures à 50 °C la suspension obtenue. On refroidit à +5 °C, ajoute 100 cm$^3$ d'éther, acidifie à l'aide d'acide chlorhydrique 2N, ajoute 50 cm$^3$ d'eau et extrait la solution obtenue à l'éther. On lave avec de l'acide chlorhydrique 2N puis avec une solution aqueuse saturée en chlorure de sodium, sèche et concentre à sec sous pression réduite. On récupère 13 g de produit attendu utilisé tel quel pour le stade suivant.

**STADE B** : 3-méthoxy alpha-méthylène benzène acétate de méthyle.

A 11 g du produit obtenu au stade A, en suspension dans 100 cm$^3$ d'eau, on ajoute 10,8 cm$^3$ de formaldéhyde à 37% dans l'eau, 8,2 g de carbonate de potassium et 5 mg d'hydroquinone. On agite 2 heures à température ambiante, extrait à l'acétate d'éthyle, lave à l'eau, sèche et évapore à sec sous pression réduite. On obtient 9,6 g de produit attendu utilisé tel quel au stade suivant.

**STADE C** : Acide 3-méthoxy alpha-méthylène benzène acétique.

On agite 5 heures à température ambiante 4,82 g de potasse en pastilles dans 121 cm$^3$ d'eau avec 9,60 g du produit obtenu au stade B dans 121 cm$^3$ de tétrahydrofuranne. On évapore le tétrahydrofuranne, extrait à l'acétate d'éthyle, lave à l'eau, réunit les phases aqueuses que l'on acidifie à l'aide d'acide chlorhydrique concentré. On extrait de nouveau à l'acétate d'éthyle, lave à l'eau, sèche et concentre à sec sous pression réduite. On obtient 9,2 g de produit attendu que l'on utilise tel quel pour le stade suivant.

**STADE D** : Acide alpha-[[(2-aminophényl) thio] méthyl] 3-méthoxy benzène acétique.

On ajoute 7,6 $cm^3$ d'aminothiophénol dans une solution comprenant 8,9 g du produit obtenu au stade C et 130 $cm^3$ d'éthanol. On agite 1 heure et demie à 95 ° C la solution obtenue, laisse revenir à température ambiante, élimine l'éthanol sous pression réduite, ajoute 50 $cm^3$ d'éther isopropylique, amorce la cristallisation, essore et sèche à 80 ° C sous pression réduite 7,2 g de produit attendu. Après chromatographie des liqueurs mères sur silice (éluant : acétate d'éthyle-cyclohexane 1-1), on recueille 710 mg de produit pur (rf = 0,16) après cristallisation dans l'éther. F = 128 ° C.

| Analyse : $C_{16}H_{17}NO_3S$ | | | | | | | | |
|---|---|---|---|---|---|---|---|---|
| Calculés<br>Trouvés | C% | 63,35<br>63,5 | H% | 5,65<br>5,5 | N% | 4,62<br>4,5 | S% | 10,57<br>10,6 |

**STADE E** : 2,3-dihydro 3-(3-méthoxyphényl) 1,5-benzothiazépin-4(5H)-one.

A 3,64 g du produit obtenu au stade D en suspension dans 110 $cm^3$ de chlorure de méthylène, on ajoute à 10 ° C en 2 minutes 4,60 g de chlorhydrate de 1-éthyl 3-(3-diméthylaminopropyl) carbodiimide. On agite 30 minutes à température ambiante, ajoute 100 $cm^3$ d'eau, extrait au chlorure de méthylène, lave à l'eau, sèche, traite au charbon actif, filtre et élimine les solvants sous pression réduite. On obtient 3,55 g de produit brut que l'on cristallise dans l'acétate d'éthyle et obtient 3,10 g de produit attendu. F = 198 ° C après recristallisation dans l'éthanol.

| Analyse : $C_{16}H_{15}NO_2S$ | | | | | | | | |
|---|---|---|---|---|---|---|---|---|
| Calculés<br>Trouvés | C% | 67,35<br>67,6 | H% | 5,30<br>5,1 | N% | 4,91<br>4,7 | S% | 11,24<br>11,3 |

**EXEMPLE 20 : Ethanedioate de (±) 2,3-dihydro 5-(2-(diméthylamino) éthyl) 3-(4-(trifluorométhyl) phényl) 1,5-benzothiazépin-4(5H)-one.**

Dans une solution comprenant 3,8 g de triphénylphosphine dans 100 $cm^3$ de tetrahydrofuranne, on ajoute en une seule fois 3,12 g de 2,3-dihydro 3-[4-(trifluorométhyl) phényl] 1,5-benzothiazépin-4(5H)-one puis 1,25 g de diméthylaminoéthanol. On agite 5 minutes puis ajoute en 10 minutes à température ambiante une solution de 2,52 g de diéthylazodicarboxylate dans 50 $cm^3$ de tétrahydrofuranne. On agite 23 heures à température ambiante, évapore le tétrahydrofuranne, obtient 13 g de produit brut que l'on chromatographie sur silice (éluant : méthanol-chlorure de méthylène 10-90). On recueille 2,2 g de produit attendu sous forme de base que l'on dissout dans 60 $cm^3$ d'isopropanol, ajoute 0,7 $cm^3$ d'acide oxalique en solution dans 20 $cm^3$ de méthanol. On concentre partiellement, amorce la cristallisation, abandonne 4 heures, essore, rince à l'isopropanol et sèche sous pression réduite à 100 ° C. On obtient 2,1 g de produit attendu. F = 179 ° C après recristallisation dans l'isopropanol.

| Analyse : $C_{22}H_{23}F_3N_2O_5S$ | | | | | | | | | | |
|---|---|---|---|---|---|---|---|---|---|---|
| Calculés<br>Trouvés | C% | 54,54<br>54,2 | H% | 4,78<br>4,7 | F% | 11,76<br>11,5 | N% | 5,78<br>5,7 | S% | 6,62<br>6,8 |

**Le 2,3-dihydro 3-[4-(trifluorométhyl) phényl] 1,5-benzothiazépin-4(5H)-one utilisé au départ a été préparé comme suit:**

**STADE A** : Acide alpha-[[(2-aminophényl) thio] méthyl] 4-(trifluorométhyl) benzène acétique.

Dans une solution comprenant 43 g d'acide alpha-méthylène 4-(trifluorométhyl) benzène acétique préparé comme indiqué dans J. Med. Chem. (1969) 12 (3) 477-80, dans 300 $cm^3$ d'éthanol, on ajoute en 1 fois, 20,41 g d'aminothiophénol et chauffe 2 heures au reflux. On évapore l'éthanol, reprend dans 600 $cm^3$

d'éther éthylique, concentre partiellement (300 $cm^3$), ajoute 200 $cm^3$ d'éther isopropylique, concentre à 100 $cm^3$, refroidit 30 minutes dans de l'eau glacée, essore le produit cristallisé, le lave à l'éther isopropylique et le sèche sous pression réduite à 100 °C. On récupère 21,9 g de produit attendu. F ≃ 138 °C.

**STADE B** : 2,3-dihydro 3-[4-(trifluorométhyl) phényl] 1,5-benzothiazépin-4(5H)-one.

On ajoute 1,74 g de 1-éthyl 3-(3-diméthylaminopropyl) carbodiimide à 1,55 g du produit obtenu au stade A en suspension dans 25 $cm^3$ de chlorure de méthylène. On agite 30 minutes, ajoute 50 $cm^3$ de chlorure de méthylène, lave à l'eau, extrait au chlorure de méthylène, sèche la phase organique et concentre à sec sous pression réduite. On recueille 1,25 g de produit attendu que l'on recristallise dans l'isopropanol. F = 195 °C.

**ETUDE PHARMACOLOGIOUE**

1) Action anti-arythmique chez le rat

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous-cutané de manière à enregistrer l'électrocardiogramme des rats sur le signal en dérivation DII.

On administre les produits à tester par voie intraveineuse ou par voie orale.

Au bout de cinq minutes dans le cas d'administration par voie intraveineuse et d'une heure dans le cas d'administration par voie orale, on perfuse la veine jugulaire des rats avec 10 microgrammes/mn sous 0,2 ml d'une solution d'aconitine et on note le temps d'apparition des premières extrasystoles ventriculaires. La quantité d'aconitine perfusée est calculée puis exprimée en fonction du poids corporel de l'animal.

Le pourcentage d'augmentation de la dose d'aconitine nécessaire pour déclencher des extrasystoles ventriculaires après traitement est calculé par rapport aux animaux témoins.

Les résultats figurant sur le tableau ci-après montrent que les produits de la présente demande sont doués de remarquables propriétés anti-arythmiques.

| Produit de l'exemple | Voie | Dose mg/kg | Pourcentage d'augmentation de la dose d'aconitine pour déclencher des extrasystoles |
|---|---|---|---|
| 11 | IV<br>PO | 5<br>25 | 32<br>135 |
| 13 | IV<br>PO | 5<br>25 | 24<br>35 |
| 6 | IV<br>PO | 5<br>25 | 142<br>> 188 |
| 7 | IV<br>PO | 5<br>25 | 25<br>129 |

2) Test de l'activité anti-agrégante plaquettaire. Agrégation plaquettaire, in vitro, sur plasma riche en plaquettes (PRP)

La mesure de l'agrégation plaquettaire est faite selon la méthode turbidimétrique de Born G.V. et Croos M.J. 1963, J. Physiol 168 178. Le sang de lapin est prélevé sur citrate de Na à 3,2 % par ponction cardiaque. Le plasma riche en plaquettes est obtenu par centrifugation et ajustement du nombre des plaquettes à 300 000 /microlitres.

L'aggrégation est induite par le collagène (40 microgrammes/ml de PRP) ou le PAF acéther (0,05 micromoles/l de PRP).

Les molécules à tester sont mises à incuber à différentes concentrations dans le PRP 2 minutes avant l'agent agrégant.

Les résultats sont exprimés en $CI_{50}$ (concentration inhibant l'agrégation de 50 % par rapport aux courbes témoins).

| Produit de l'exemple | $CI_{50}$ en $10^{-5}$ moles/1 | |
|---|---|---|
| | Induction par le COLLAGENE | Induction par le PAF-ACETHER |
| 11 | 0,8 | 4 |
| 12 | 5,6 | 0,9 |
| 1 | 7,4 | 7,8 |
| 9 | 6,6 | 5,8 |

3) Test de l'activité anti-sérotoninergique.

Bronchoconstriction, chez le cobaye, induite par la sérotonine.

La résistance pulmonaire à l'insufflation est mesurée par la technique de "l'overflow" selon KONZETT H., Rossler R., Arch. Ex. Pathol. Pharmakol, 1940 195 71.

Les cobayes sont anesthésiés à l'uréthane, la jugulaire est cathétérisée pour injecter les produits, la trachée pour installer la ventilation artificielle.

La bronchoconstriction est induite par une dose de 3 à 5 microgrammes/kg de sérotonine injectée par voie intraveineuse.

Les antagonistes sont injectés une minute avant l'induction du bronchospasme.

On constate d'après les résultats figurant sur le tableau ci-après que les produits de la présente demande ont une forte activité anti-sérotoninergique.

| Produit de l'exemple | Dose mg/kg | Pourcentage d'inhibition de la bronchoconstriction |
|---|---|---|
| 11 | 1 | 82 |
| 12 | 1 | 64 |
| 13 | 1 | 87 |
| 1 | 1 | 79 |
| 5 | 1 | 87 |

4) Etude de la toxicité aigüe

On a évalué les doses létales DL des différents composés testés après 1 seule administration par voie intrapéritonéale ou orale chez la souris.

On appelle $DL_0$ la dose maximale ne provoquant aucune mortalité en 8 jours.

Les résultats obtenus sont les suivants :

| Produit de l'exemple | $DL_0$ en mg/kg | |
|---|---|---|
| | IP | PO |
| 11 | 80 | 100 |
| 12 | 100 | > 200 |
| 13 | 80 | > 200 |
| 6 | 100 | 100 |
| 7 | 100 | 100 |

**Revendications**

**1.** Les produits de formule générale (I) :

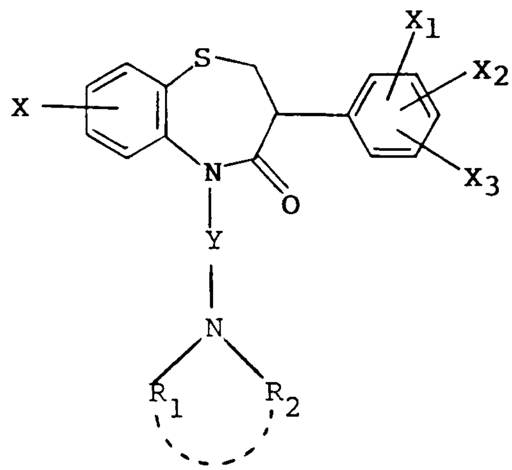

(I)

dans laquelle X représente un atome d'hydrogène, un atome d'halogène, un radical hydroxyle, un radical alkyle ou alkyloxy, renfermant de 1 à 3 atomes de carbone, un radical nitro, amino, monoalkyle ou dialkylamino, ou un radical trifluorométhyle,

$X_1$, $X_2$ et $X_3$ identiques ou différents représentent un atome d'hydrogène, un atome d'halogène, un radical hydroxyle, un radical alkyle ou alkyloxy renfermant de 1 à 3 atomes de carbone, un radical nitro, amino, monoalkyle ou dialkylamino, un radical trifluorométhyle, trifluorométhylthio ou trifluorométhoxy,

Y représente soit le groupement

$$-(CH_2)_r-\underset{R_4}{\overset{R_3}{C}}-(CH_2)_s-$$

dans lequel

r et s identiques ou différents sont des entiers de 0 à 4, étant entendu que la somme de r et s doit être un entier de 1 à 4,

$R_3$ et $R_4$ identiques ou différents représentent un atome d'hydrogène ou un radical alkyle renfermant de 1 à 4 atomes de carbone,

soit le groupement

$$-(CH_2)_{r1}-\underset{\underset{O}{\|}}{C}-$$

dans lequel r1 représente un entier de 1 à 4,

$R_1$ et $R_2$ identiques ou différents représentent un atome d'hydrogène ou un radical alkyle renfermant de 1 à 4 atomes de carbone éventuellement substitué soit par un radical aryle renfermant de 6 à 12 atomes de carbone lui-même éventuellement substitué par l, 2 ou 3 radicaux choisis parmi les radicaux alkyle, alkyloxy renfermant de 1 à 4 atomes de carbone, hydroxy, cyano et halogène, soit par une amine éventuellement mono ou disubstituée par un radical alkyle renfermant de 1 à 4 atomes de carbone, ou $R_1$ et $R_2$ forment avec l'atome d'azote auquel ils sont liés un radical hétérocyclique saturé à cinq, six ou sept chaînons comprenant éventuellement un autre hétéroatome choisi parmi les atomes d'azote, d'oxygène ou de soufre et éventuellement substitué,

lesdits composés de formule (I) pouvant être sous toutes les formes isomères possibles, racémique ou optiquement actives ainsi que les sels d'addition avec les acides minéraux ou organiques.

**2.** Les produits de formule générale (I) tels que définis à la revendication 1, dans laquelle X représente un atome d'hydrogène ainsi que leurs sels d'addition avec les acides minéraux ou organiques.

**3.** Les produits de formule générale (I), tels que définis aux revendications 1 et 2 dans laquelle $R_1$ et $R_2$ représentent chacun un radical méthyle, éthyle, isopropyle, ceux dans lesquels $R_1$ représente un radical méthyle et $R_2$ représente un radical phénéthyle éventuellement mono ou di substitué par un radical méthyle ou méthoxy, ceux dans lesquels $R_1$ représente un atome d'hydrogène et $R_2$ représente un radical méthyle ou éthyle substitué par une dialkylamine, ou $R_1$ et $R_2$ forment avec l'atome d'azote auquel ils sont liés un radical pyrrolidinyle, pipéridyle, morpholinyle ou pipérazinyle éventuellement substitué sur l'atome d'azote non lié au radical $(CH_2)_s$- par un radical alkyle renfermant de 1 à 4 atomes de carbone ou par un radical aryle ou arylalkyle renfermant de 6 à 12 atomes de carbone lui-même éventuellement substitué par un atome d'halogène ou un radical alkyle ou alkyloxy renfermant de 1 à 4 atomes de carbone ainsi que leurs sels d'addition avec les acides minéraux ou organiques.

**4.** Les produits de formule générale (I) tels que définis aux revendications 1, 2 et 3, dans laquelle $X_1$ et $X_2$ identiques ou différents représentent un atome de chlore ou de brome ou un radical hydroxy ou méthoxy et $X_3$ représente un atome d'hydrogène ainsi que leurs sels d'addition avec les acides minéraux ou organiques.

**5.** Les produits de formule générale (I), tels que définis à l'une quelconque des revendications 1 à 4, dans laquelle Y représente le groupement

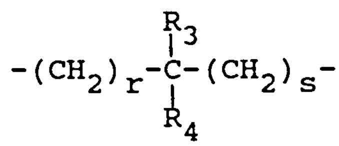

ainsi que leurs sels d'adition avec les acides minéraux ou organiques.

**6.** Les produits de formule générale (I) selon la revendication 5, dans laquelle la somme r + s est égale à 1 ou 2, et $R_3$ et $R_4$ représentent chacun un atome d'hydrogène ainsi que leurs sels d'addition avec les acides minéraux ou organiques.

**7.** Les produits de formule générale (I) telle que définie à l'une quelconque des revendications 1 à 4, dans laquelle Y représente le groupement

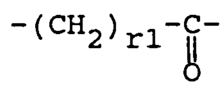

dans lequel r1 représente le nombre 1 ainsi que leurs sels d'addition avec les acides, minéraux ou organiques.

**8.** Les dérivés de formule générale (I) telle que définie à l'une quelconque des revendications 1 à 6, dont les noms suivent :

- le (±)-5-[2-(diméthylamino) éthyl] 2,3-dihydro 3-(4-chloro phényl) 1,5-benzothiazépin-4(5H)-one,
- le (±)-5-[2-(diméthylamino) éthyl] 2,3-dihydro 3-(2-méthoxy phényl) 1,5-benzothiazépin-4(5H)-one,
- le (±)-5-[2-(diméthylamino) éthyl] 2,3-dihydro 3-(4-méthoxy phényl) 1,5-benzothiazépin-4(5H)-one,

ainsi que leurs sels d'addition avec les acides.

**9.** Procédé de préparation des produits de formule (I) telle que définie à la revendication 1, caractérisé en ce que :

1) pour préparer des produits de formule (I) dans laquelle Y représente le groupe

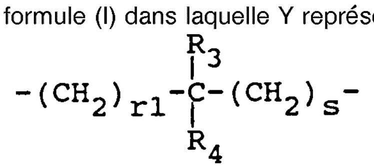

l'on traite un produit de formule (II) :

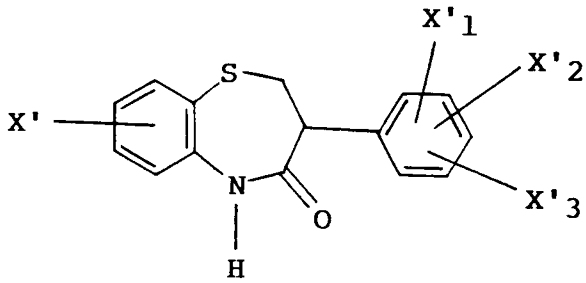

(II)

sous forme racémique ou optiquement active dans laquelle X′ a la signification de X, telle que définie précédemment ou bien X′ représente un radical réactif protégé, et $X'_1$, $X'_2$ et $X'_3$ identiques ou différents ont la signification de $X_1$, $X_2$ et $X_3$ telle que définie à la revendication 1 ou bien représentent un radical réactif protégé :

- soit par un produit de formule (III) :

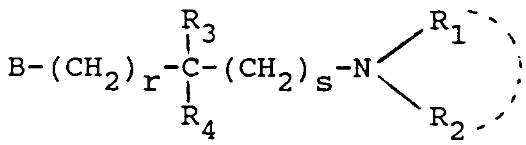

(III)

dans laquelle $R_1$, $R_2$, $R_3$, $R_4$, r et s ont les significations indiquées à la revendication 1 et B représente un atome d'halogène ou un radical hydroxyle, pour obtenir le produit de formule (X) :

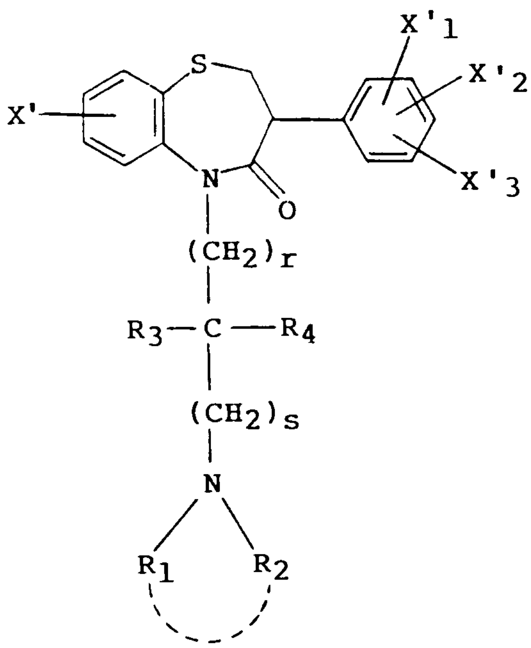

(X)

- soit, pour obtenir un produit de formule (I) dans laquelle s représente $s_1$, $s_1$ étant un entier de 1 à 4, par un produit de formule (IV) :

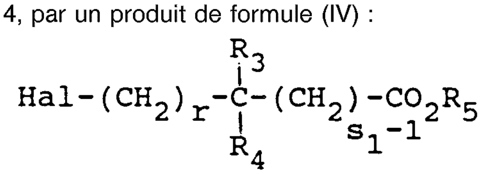

(IV)

dans laquelle Hal représente un atome d'halogène, $R_3$, $R_4$ et r ont les significations indiquées à la revendication 1,

$s_1$ a la signification indiquée ci-dessus,
$R_5$ représente un radical alkyle renfermant de 1 à 4 atomes de carbone,
pour obtenir un produit de formule (V) :

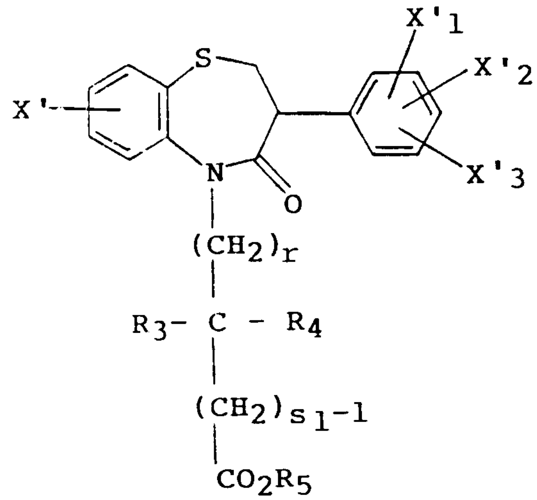

(V)

que l'on soumet à une réaction d'hydrolyse pour obtenir le composé de formule (VI) :

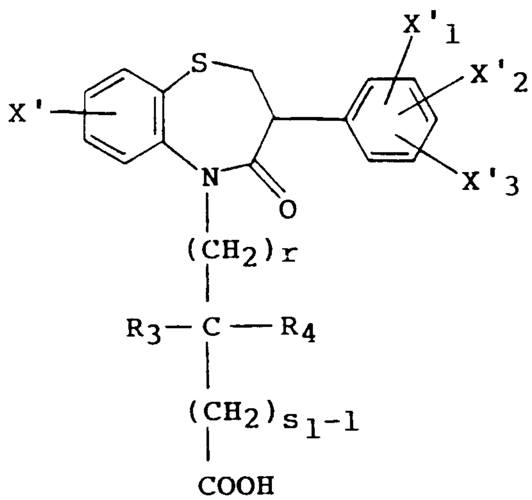

(VI)

que l'on soumet à une réaction de réduction pour obtenir un produit de formule (VII) :

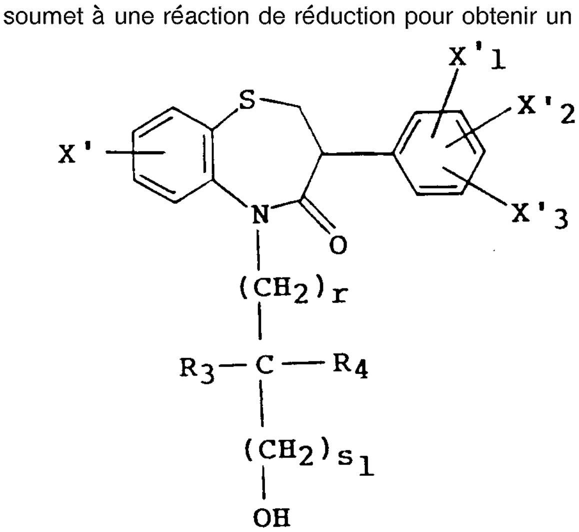

(VII)

que l'on soumet à une réaction de substitution par un atome d'halogène Hal pour obtenir un produit

de formule (VIII) :

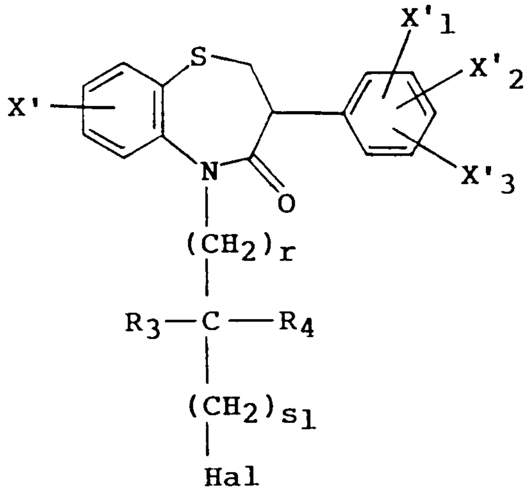

(VIII)

que l'on fait réagir avec l'amine de formule (IX) :

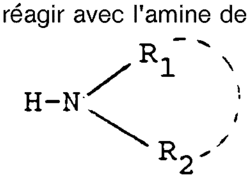

(IX)

pour obtenir un produit de formule (XI) :

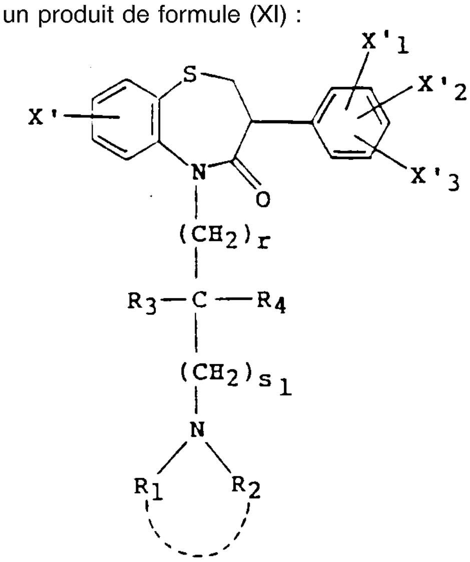

(XI)

2) pour préparer des produits de formule (I) dans laquelle Y représente un groupe

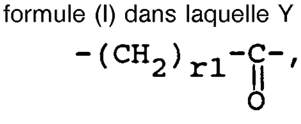

l'on traite un produit de formule (II) telle que définie ci-dessus <u>soit</u> par un produit de formule (III') :

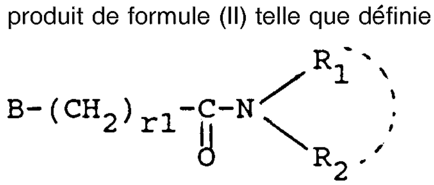

(III')

dans laquelle B, r1, $R_1$ et $R_2$ ont la signification indiquée précédemment pour obtenir un produit de formule (XVII) :

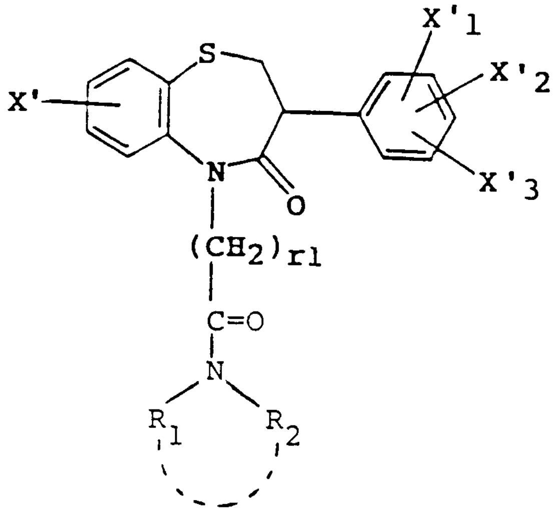

(XVII)

<u>soit</u> par un produit de formule (XVIII) :

$$Hal-(CH_2)_{r1}-\underset{\underset{O}{\|}}{C}-OR_5 \qquad (XVIII)$$

dans laquelle $R_5$ et r1 ont les valeurs indiquées précédemment pour obtenir un produit de formule (XIX) :

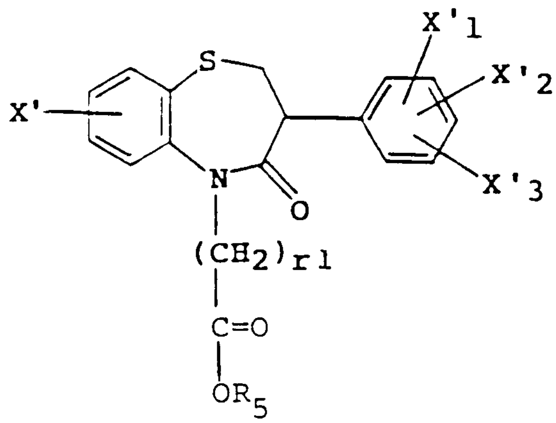

(XiX)

que l'on soumet à une réaction d'hydrolyse pour obtenir le composé de formule (XX) :

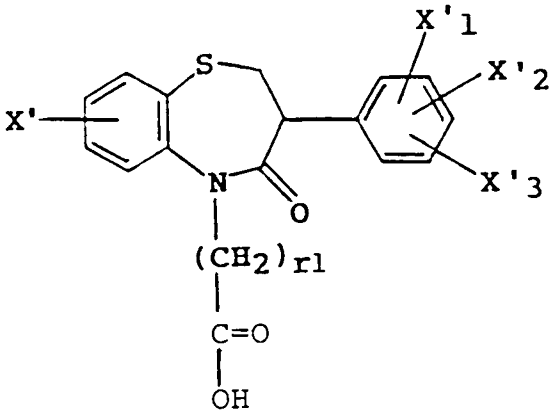

(XX)

que l'on fait réagir avec une amine de formule (IX) telle que définie ci-dessus pour obtenir un produit de formule (XVII) telle que définie ci-dessus, puis soumet si nécessaire ou si désiré les produits de formule (X), (XI) et (XVII) obtenus sous forme racémique ou optiquement active, à l'une ou plusieurs des réactions suivantes, dans un ordre quelconque :

a) coupure par hydrolyse ou hydrogénolyse du groupe protecteur du ou des radicaux réactifs protégés,

b) dédoublement des produits racémiques par des procédés classiques pour obtenir les produits optiquement actifs,

c) salification pour obtenir les sels correspondants.

**10.** A titre de médicaments, les produits de formule (I) tels que définis à l'une quelconque des revendications 1 à 7 ainsi que leurs sels d'addition avec les acides pharmaceutiquement acceptables.

**11.** A titre de médicaments les produits de la revendication 8 ainsi que leurs sels d'addition avec les acides pharmaceutiquement acceptables.

**12.** Les compositions pharmaceutiques contenant à titre de principe actif l'un au moins des médicaments tels que définis par les revendications 10 et 11.

**13.** A titre de produits industriels nouveaux, les produits intermédiaires répondant aux formules (V), (VI), (VII), (VIII), (XIX) et (XX) tels que définis à la revendication 9.

## Claims

**1.** The products of general formula (I):

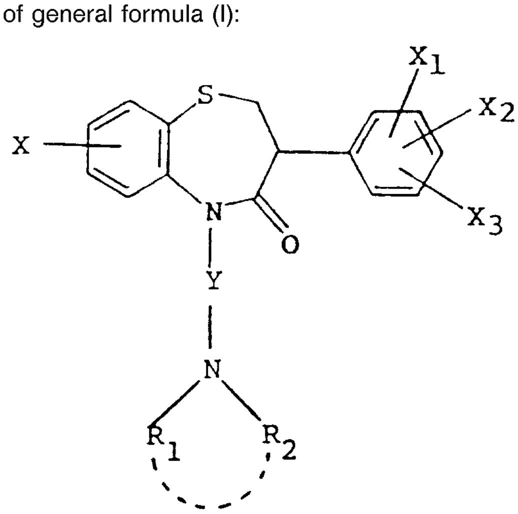

(I)

in which X represents a hydrogen atom, a halogen atom, a hydroxyl radical, an alkyl or alkyloxy radical

containing 1 to 3 carbon atoms, a nitro, amino, monoalkyl or dialkylamino radical, or a trifluoromethyl radical,
$X_1$, $X_2$ and $X_3$ identical or different represent a hydrogen atom, a halogen atom, a hydroxyl radical, an alkyl or alkyloxy radical containing 1 to 3 carbon atoms, a nitro, amino, monoalkyl or dialkylamino radical, a trifluoromethyl, trifluoromethylthio or trifluoromethoxy radical,
Y represents either the

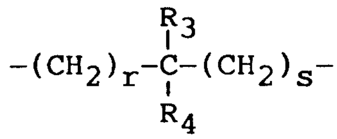

group in which
r and s identical or different are integers from 0 to 4, it being understood that the sum of r and s must be an integer from 1 to 4,
$R_3$ and $R_4$ identical or different represent a hydrogen atom or an alkyl radical containing 1 to 4 carbon atoms,
or the

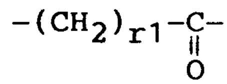

group in which r1 represents an integer from 1 to 4,
$R_1$ and $R_2$ identical or different represent a hydrogen atom or an alkyl radical containing 1 to 4 carbon atoms optionally substituted either by an aryl radical containing 6 to 12 carbon atoms itself optionally substituted by 1, 2 or 3 radicals chosen from alkyl, alkyloxy radicals containing 1 to 4 carbon atoms, hydroxy, cyano and halogen, or by an amine optionally mono- or disubstituted by an alkyl radical containing 1 to 4 carbon atoms, or $R_1$ and $R_2$ form with the nitrogen atom to which they are linked a saturated heterocyclic radical with five, six or seven members optionally containing another heteroatom chosen from nitrogen, oxygen or sulphur atoms and optionally substituted, the said compounds of formula (I) being in all possible racemic or optically active isomer forms as well as the addition salts with mineral or organic acids.

2. The products of general formula (I) as defined in claim 1, in which X represents a hydrogen atom as well as their addition salts with mineral or organic acids.

3. The products of general formula (I), as defined in claims 1 and 2 in which $R_1$ and $R_2$ each represent a methyl, ethyl, isopropyl radical, those in which $R_1$ represents a methyl radical and $R_2$ represents a phenethyl radical optionally mono- or di- substituted by a methyl or methoxy radical, those in which $R_1$ represents a hydrogen atom and $R_2$ represents a methyl or ethyl radical substituted by a dialkylamine, or $R_1$ and $R_2$ form with the nitrogen atom to which they are linked a pyrrolidinyl, piperidyl, morpholinyl or piperazinyl radical optionally substituted on the nitrogen atom which is not linked to the $(CH_2)_s$-radical by an alkyl radical containing 1 to 4 carbon atoms or by an aryl or arylalkyl radical containing 6 to 12 carbon atoms itself optionally substituted by a halogen atom or an alkyl or alkyloxy radical containing 1 to 4 carbon atoms as well as their addition salts with mineral or organic acids.

4. The products of general formula (I) as defined in claims 1, 2 and 3, in which $X_1$ and $X_2$ identical or different represent a chlorine or bromine atom or a hydroxy or methoxy radical and $X_3$ represents a hydrogen atom as well as their addition salts with mineral or organic acids.

5. The products of general formula (I), as defined in any one of claims 1 to 4, in which Y represents the

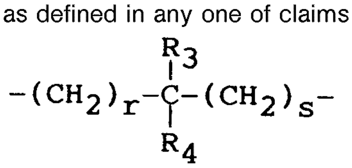

group as well as their addition salts with mineral or organic acids.

**6.** The products of general formula (I) according to claim 5, in which the sum r + s is equal to 1 or 2, and $R_3$ and $R_4$ each represent a hydrogen atom as well as their addition salts with mineral or organic acids.

**7.** The products of general formula (I) as defined in any one of claims 1 to 4, in which Y represents the

$$-(CH_2)_{r1}-\underset{\displaystyle O}{C}-$$

group in which r1 represents the number 1 as well as their addition salts with mineral or organic acids.

**8.** The derivatives of general formula (I) as defined in any one of claims 1 to 6, the names of which follow:
- (±)-5-[2-(dimethylamino) ethyl] 2,3-dihydro 3-(4-chloro phenyl) 1,5-benzothiazepin-4(5H)-one,
- (±)-5-[2-(dimethylamino) ethyl] 2,3-dihydro 3-(2-methoxy phenyl) 1,5-benzothiazepin-4(5H)-one,
- (±)-5-[2-(dimethylamino) ethyl] 2,3-dihydro 3-(4-methoxy phenyl) 1,5-benzothiazepin-4(5H)-one,

as well as their addition salts with acids.

**9.** Preparation process for the products of formula (I) as defined in claim 1, characterized in that:
1) to prepare the products of formula (I) in which Y represents the

$$-(CH_2)_{r1}-\overset{\displaystyle R_3}{\underset{\displaystyle R_4}{C}}-(CH_2)_s-$$

group a product of formula

(II):

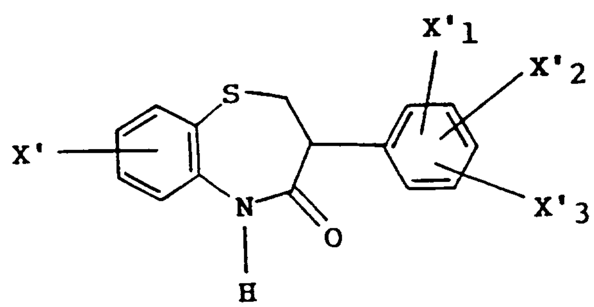

(II)

in racemic or optically active form in which X' has the meaning of X, as defined previously, or X' represents a protected reactive radical, and $X'_1$, $X'_2$ and $X'_3$ identical or different have the meaning of $X_1$, $X_2$ and $X_3$ as defined in claim 1 or represent a protected reactive radical, is treated:
- either with a product of formula (III):

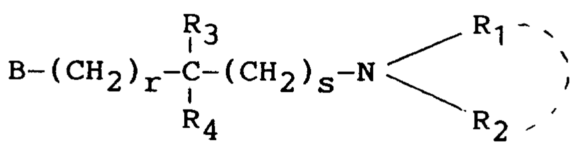

(III)

in which $R_1$, $R_2$, $R_3$, $R_4$, r and s have the meanings indicated in claim 1 and B represents a halogen atom or a hydroxyl radical, in order to obtain the product of formula (X):

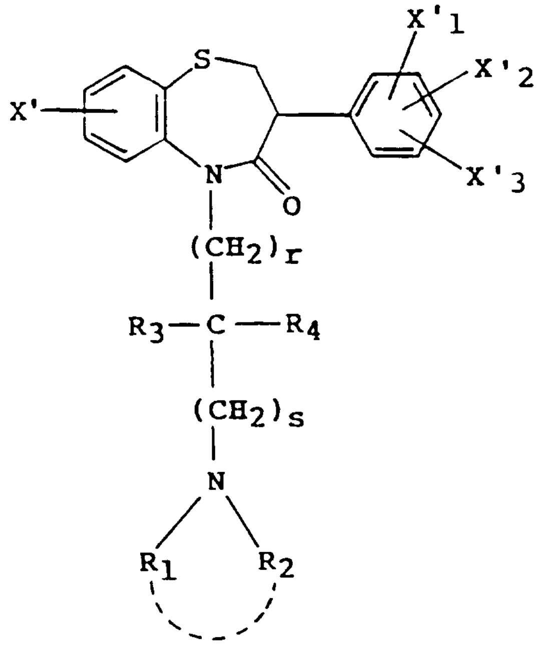

(X)

- or, to obtain a product of formula (I) in which s represents $s_1$, $s_1$ being an integer from 1 to 4, with a product of formula (IV):

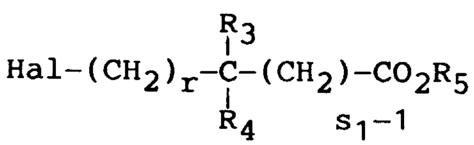

(IV)

in which Hal represents a halogen atom, $R_3$, $R_4$ and r have the meanings indicated in claim 1, $s_1$ has the meaning indicated above,
$R_5$ represents an alkyl radical containing 1 to 4 carbon atoms,
in order to obtain a product of formula (V):

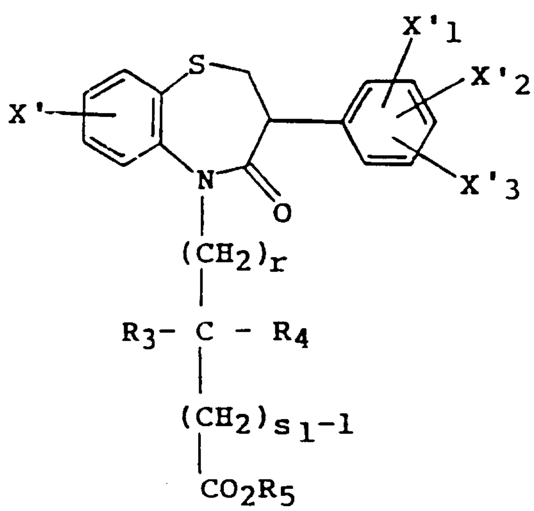

(V)

which is subjected to a hydrolysis reaction in order to obtain the compound of formula (VI):

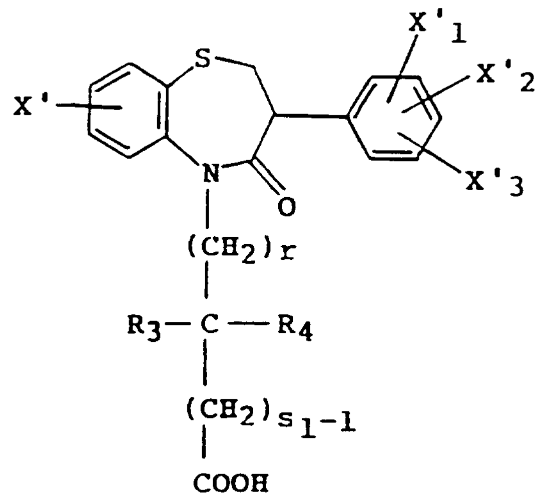

(VI)

which is subjected to a reduction reaction in order to obtain a product of formula (VII):

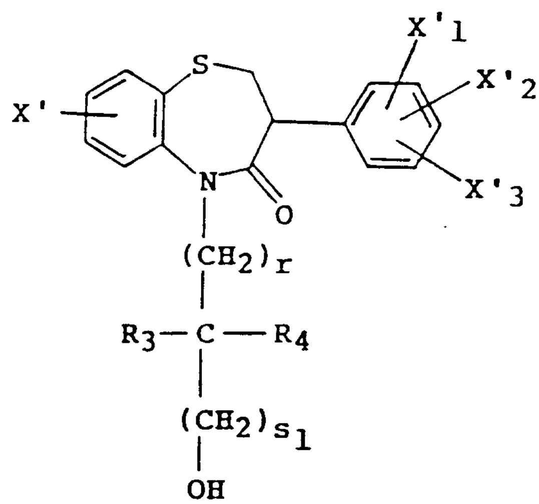

(VII)

which is subjected to a substitution reaction by a halogen atom Hal in order to obtain a product of formula (VIII):

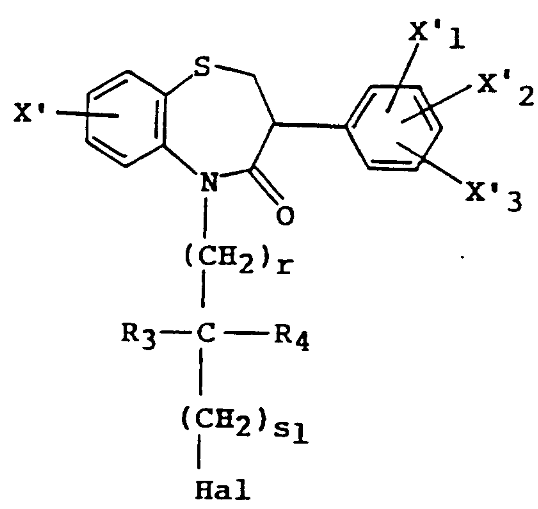

(VIII)

which is reacted with the amine of formula (IX):

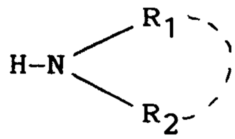

(IX)

in order to obtain a product of formula (XI):

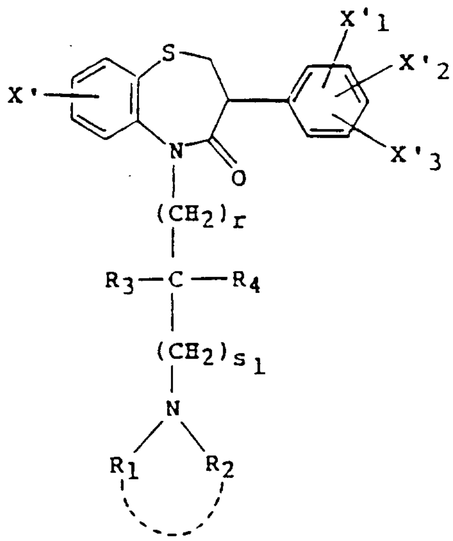

(XI)

2) to prepare the products of formula (I) in which Y represents a

$$-(CH_2)_{r1}-\underset{\displaystyle O}{C}-$$

group, a product of formula (II) as defined above is treated <u>either</u> with a product of formula (III'):

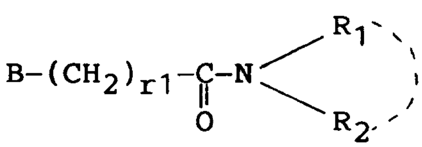

(III')

in which B, r1, $R_1$ and $R_2$ have the meaning indicated previously in order to obtain a product of formula (XVII):

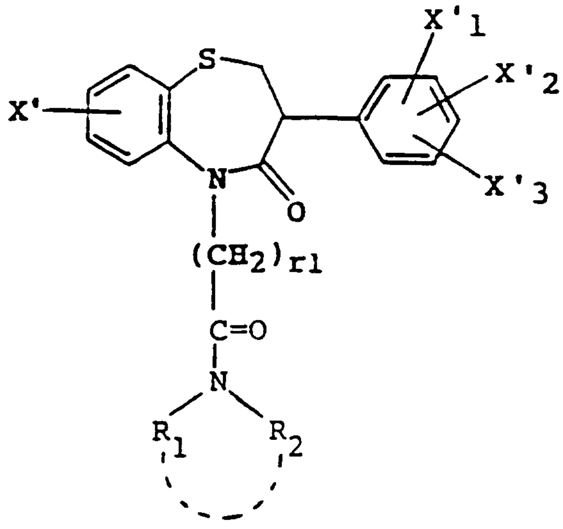

(XVII)

or with a product of formula (XVIII):

$$Hal-(CH_2)_{r1}-\underset{\underset{O}{\|}}{C}-OR_5 \qquad (XVIII)$$

in which $R_5$ and r1 have the values indicated previously in order to obtain a product of formula (XIX):

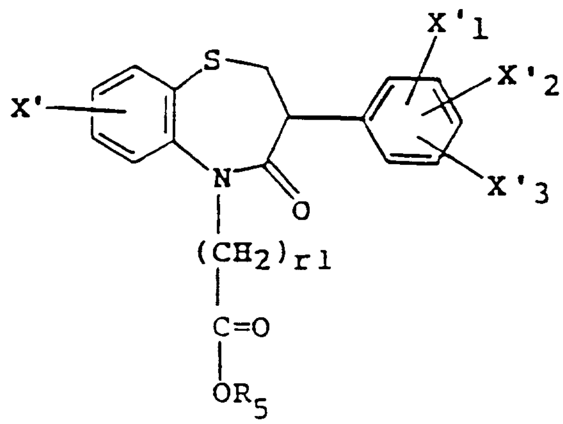

(XIX)

which is subjected to a hydrolysis reaction in order to obtain the compound of formula (XX):

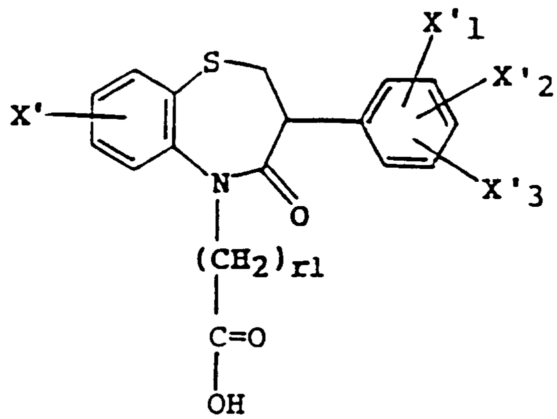

(XX)

which is reacted with an amine of formula (IX) as defined above in order to obtain a product of formula (XVII) as defined above, then if necessary or if desired the products of formulae (X), (XI) and (XVII) obtained in racemic or optically active form, are subjected to one or more of the following reactions, in any order:

a) cutting by hydrolysis or hydrogenolysis of the protective group of the protected reactive radical or radicals,

b) resolution of the racemic products by standard processes in order to obtain the optically active products,

c) salification in order to obtain the corresponding salts.

**10.** As medicaments, the products of formula (I) as defined in any one of claims 1 to 7 as well as their addition salts with pharmaceutically acceptable acids.

**11.** As medicaments the products of claim 8 as well as their addition salts with pharmaceutically acceptable acids.

**12.** The pharmaceutical compositions containing at least one of the medicaments as defined by claims 10 and 11 as active ingredient.

**13.** As new industrial products, the intermediate products corresponding to formulae (V), (VI), (VII), (VIII), (XIX) and (XX) as defined in claim 9.

**Patentansprüche**

**1.** Die Produkte mit der allgemeinen Formel (I):

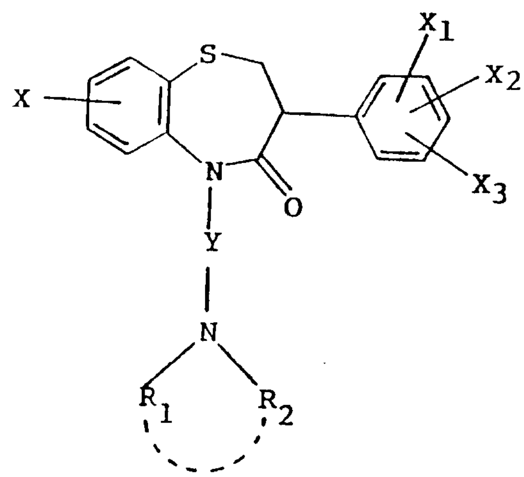

(I)

in der X für ein Wasserstoffatom, ein Halogenatom, einen Hydroxylrest, einen Alkyl- oder Alkoxyrest mit 1 bis 3 Kohlenstoffatomen, eine Nitro-, Amino-, Monoalkyl- oder Dialkylaminogruppe oder einen Trifluormethylrest steht,

$X_1$, $X_2$ und $X_3$, gleich oder verschieden, für ein Wasserstoffatom, ein Halogenatom, einen Hydroxylrest, einen Alkyl- oder Alkoxyrest mit 1 bis 3 Kohlenstoffatomeil, eine Nitro-, Amino-, Monoalkyl- oder Dialkylaminogruppe, einen Tiifluormethyl-, Trifluormethylthio- oder Trifluormethoxyrest stehen,

Y entweder für die Gruppierung

$$-(CH_2)_r-\underset{R_4}{\overset{R_3}{\underset{|}{\overset{|}{C}}}}-(CH_2)_s-$$

steht, in der r und s,

gleich oder verschieden, ganze Zahlen von 0 bis 4 sind, wobei die Sumnie von r und s eine ganze Zahl

von 0 bis 4 sein muß,
$R_3$ und $R_4$, gleich oder verschieden, für ein Wasserstoffatom oder für einen Alkylrest mit 1 bis 4 Kohlenstoffatomen stehen,
oder für die Gruppierung

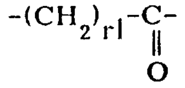 ,

in der r1 für eine ganze Zahl von 1 bis 4 steht,
$R_1$ und $R_2$, gleich oder verschieden, für ein Wasserstoffatom oder einen Alkylrest mit 1 bis 4 Kohlenstoffatomen stehen, der entweder mit einem Arylrest mit 6 bis 12 Kohlenstoffatomen substituiert ist, der wiederum selbst gegebenenfalls mit 1, 2 oder 3 Resten substituiert ist, ausgewählt aus folgenden Resten: Alkyl-, Alkoxyresten mit 1 bis 4 Kohlenstoffatomen, Hydroxyl-, Cyano- und Halogenrest, oder mit einem Amin, das gegebenenfalls ein- oder zweifach mit einen, Alkylrest mit 1 bis 4 Kohlenstoffatomen substituiert ist, oder $R_1$ und $R_2$ zusammen mit dem Stickstoffatom, an das sie gebunden sind, einen gesättigten heterozyklischen Rest mit fünf, sechs oder sieben Gliedern bilden, der gegebenenfalls ein anderes Heteroatom enthält, ausgewählt aus dem Stickstoff- Sauerstoff-oder Schwefelatom und gegebenenfalls substituiert,
wobei besagte Verbindungen nach Formel (I) in allen möglichen isomeren Formen vorkommen können, als Racemat oder optisch aktive Substanzen, sowie die Additionssalze mit den Mineralsäuren oder organischen Säuren.

**2.** Die Produkte mit der allgemeinen Formel (I), wie sie in Anspruch 1 definiert sind, in der X für ein Wasserstoffatom steht, sowie ihre Additionssalze mit den Mineralsäuren oder organischen Säuren.

**3.** Die Produkte mit der allgemeinen Formel (I), wie sie in den Ansprüchen 1 und 2 definiert sind, in der $R_1$ und $R_2$ jeweils für einen Methyl-, Ethyl-, Isopropylrest stehen, diejenigen, in denen $R_1$ für einen Methylrest und $R_2$ für einen Phenethylrest stehen, der gegebenenfalls ein- oder zweifach substituiert ist mit einem Methyl- oder Methoxyrest, diejenigen, in denen $R_1$ für ein Wasserstoffatom steht und $R_2$ für einen Methyl- oder Ethylrest, substituiert mit einem Dialkylamin steht, oder $R_1$ und $R_2$ mit dem Stickstoffatom, an das sie gebunden sind, einen Pyrrolidinyl-, Piperidinyl-Morpholinyl-oder Piperazinylrest bilden, gegebenenfalls substituiert am Stickstoffatom, das nicht an den $(CH_2)_s$- Rest gebunden ist, und zwar mit einen, Alkylrest mit 1 bis 4 Kohlenstoffatomen oder mit einem Aryl-oder Arylalkylrest mit 6 bis 12 Kohlenstoffatomen, der selbst wiederum gegebenenfalls mit einem Halogenatom oder einem Alkyl- oder Alkoxyrest mit 1 bis 4 Kohlenstoffatomen substituiert ist, sowie ihre Additionssalze mit den Mineralsäuren oder organischen Säuren.

**4.** Die Produkte mit der allgemeinen Formel (I), wie sie in den Ansprüchen 1, 2 und 3 definiert sind, in der $X_1$ und $X_2$, gleich oder verschieden, für ein Chlor- oder Bromatom stehen oder für einen Hydroxyl- oder Methoxyrest und $X_3$ für ein Wasserstoffatom steht, sowie ihre Additionssalze mit den Mineralsäuren oder organischen Säuren.

**5.** Die Produkte mit der allgemeinen Formel (I), wie sie in irgendeinem der Ansprüche 1 bis 4 definiert sind, in der Y für die Gruppierung

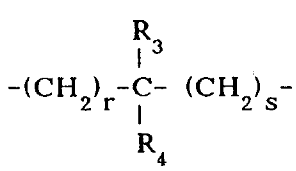

steht, sowie ihre Additionssalze mit den Mineralsäuren oder organischen Säuren.

**6.** Die Produkte mit der allgemeinen Formel (I) nach Anspruch 5, in der die Summe r + s gleich 1 oder 2 ist und $R_3$ und $R_4$ jeweils für ein Wasserstoffatom stellen, sowie ihre Additionssalze mit den Mineralsäuren oder organischen Säuren.

**7.** Die Produkte mit der allgemeinen Formel (I), wie sie in irgendeinem der Ansprüche 1 bis 4 definiert ist, in der Y für die Gruppierung

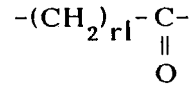

steht, in der r1 für die Zahl 1 steht, sowie ihre Additionssalze mit den Mineralsäuren oder organischen Säuren.

**8.** Die Derivate mit der allgemeinen Formel (I) wie sie in irgendeinem der Ansprüche 1 bis 6 definiert ist, mit folgenden Namen:

- das (±)-5-[2-(Dimethylamino)ethyl]-2,3-dihydro-3-(4-chlorphenyl)-1,5-benzothiazepin-4(5H)-on,
- das (±)-5-[2-(Dimethylamino)ethyl]-2,3-dihydro-3-(2-methoxyphenyl)-1,5-benzothiazepin-4(5H)-on,
- das (±)-5-[2-(Dimethylamino)ethyl]-2,3-dihydro-3-(4-methoxyphenyl)-1,5-benzothiazepin-4(5H)-on,

sowie ihre Additionssalze mit den Säuren.

**9.** Verfahren zur Herstellung der Produkte nach Formel (I), wie sie in Anspruch 1 definiert ist, dadurch gekennzeichnet, daß man

1) um Produkte mit der Formel (I) herzustellen, in der Y für die Gruppierung

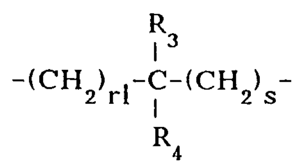

steht, ein Produkt mit der Formel (II):

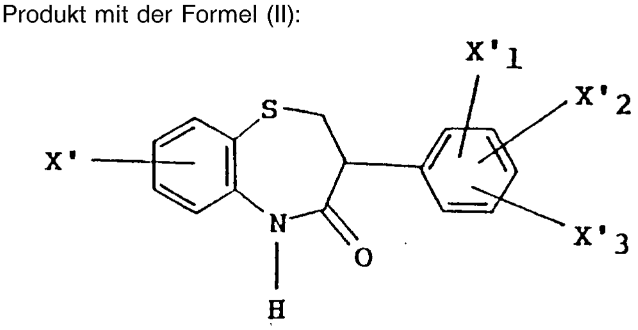

(II)

in racemischer oder optisch aktiver Form, in der X' die Bedeutung von X hat, wie sie vorher definiert ist, oder aber X' für einen geschützten reaktiven Rest steht, und $X'_1$, $X'_2$ und $X'_3$, gleich oder verschieden, die Bedeutung von $X_1$, $X_2$ und $X_3$ haben, wie sie in Anspruch 1 definiert ist oder aber für einen geschützten Rest stehen,

- entweder mit einem Produkt mit der Formel (III) behandelt:

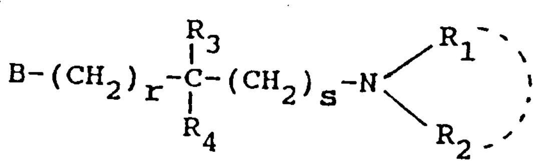

(III)

in der $R_1$, $R_2$, $R_3$, $R_4$, r und s die in Anspruch 1 angegebenen Bedeutungen haben und B für ein Halogenatom oder einen Hydroxylrest steht, wodurch man das Produkt mit der Formel (X) erhält:

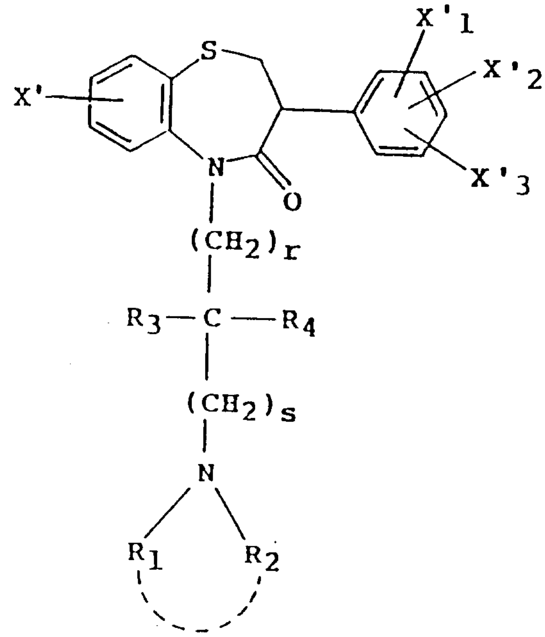

(X)

- oder man behandelt es, uni ein Produkt mit der Formel (I) zu erhalten, in der s für $s_1$ steht, wobei $s_1$ eine ganze Zahl von 1 bis 4 ist, mit einem Produkt mit der Formel (IV):

$$Hal - (CH_2)_r - \underset{R_4}{\overset{R_3}{C}} - (CH_2)_{s_1-1}CO_2R_5 \qquad (IV)$$

in der Hal für ein Halogenatom steht, $R_3$, $R_4$ und r die in Anspruch 1 angegebene Bedeutung haben,

$s_1$ die oben angegebene Bedeutung hat,

$R_5$ für einen Alkylrest steht, der 1 bis 4 Kohlenstoffatome enthält, wodurch man ein Produkt mit der Formel (V) erhält:

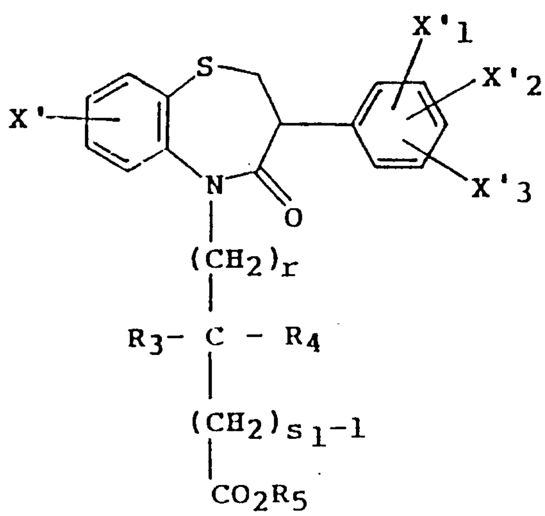

(V)

das man einer Hydrolyse unterwirft, wodurch man die Verbindung mit der Formel (VI) erhält:

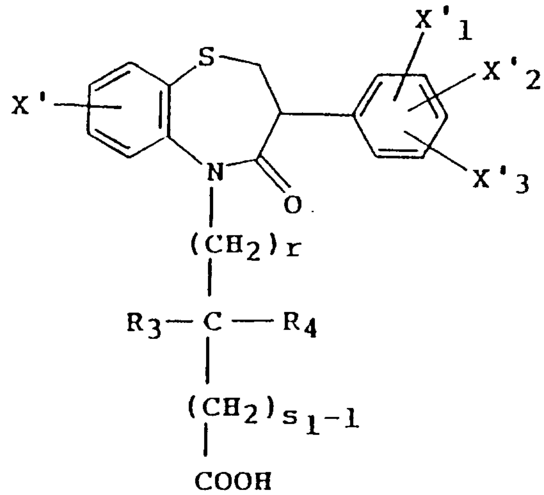

(VI)

die man einer Reduktion unterwirft, wodurch man ein Produkt mit der Formel (VII) erhält:

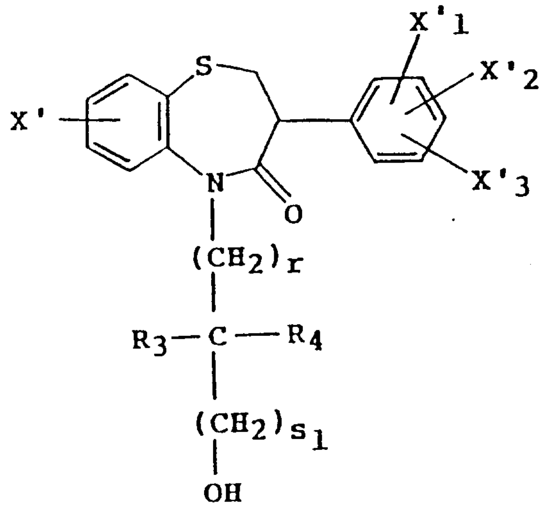

(VII)

das man einer Substitutionsreaktion durch ein Halogenatom Hal unterwirft, wodurch man ein Produkt mit der Formel (VIII) erhält:

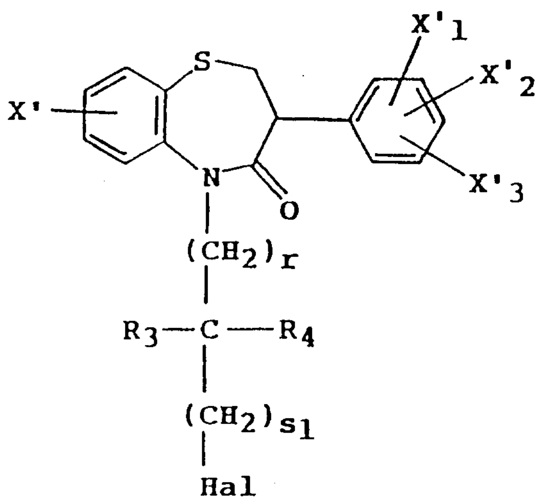

(VIII)

das man mit einem Amin mit der Formel (IX) reagieren läßt:

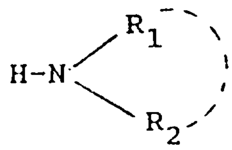

(IX)

wodurch man ein Produkt mit der Formel (XI) erhält:

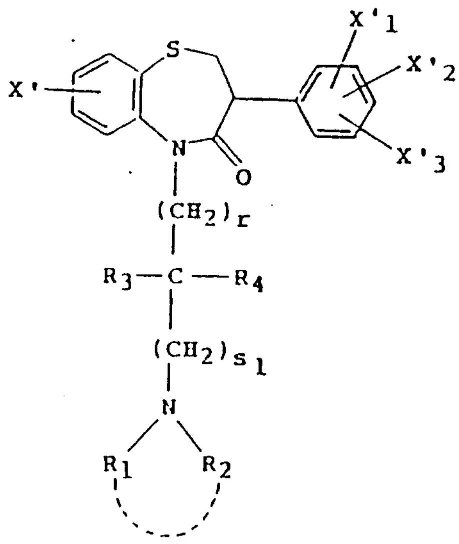

(XI)

2) daß man, um Produkte mit der Formel (I) herzustellen, in der Y für ein Gruppierung

$$-(CH_2)_{r1}-\underset{\underset{O}{\|}}{C}-$$

steht, ein Produkt mit der Formel (II), wie sie oben definiert ist, <u>entweder</u> mit einem Produkt mit der Formel (III') behandelt:

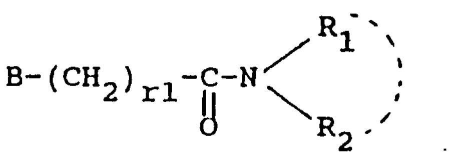

(III')

in der B, r1, $R_1$ und $R_2$ die vorher angegebene Bedeutung haben, wodurch man ein Produkt mit der Formel (XVII) erhält:

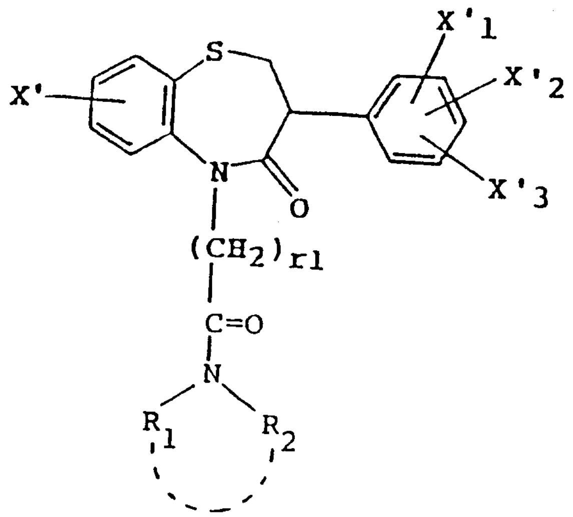

(XVII)

oder man behandelt es mit einem Produkt mit der Formel (XVIII):

$$Hal - (CH_2)_{r1} - \underset{\underset{O}{\|}}{C} - OR_5 \qquad (XVIII)$$

in der $R_5$ und r1 die vorher angegebenen Werte haben, wodurch man ein Produkt mit der Formel (XIX) erhält:

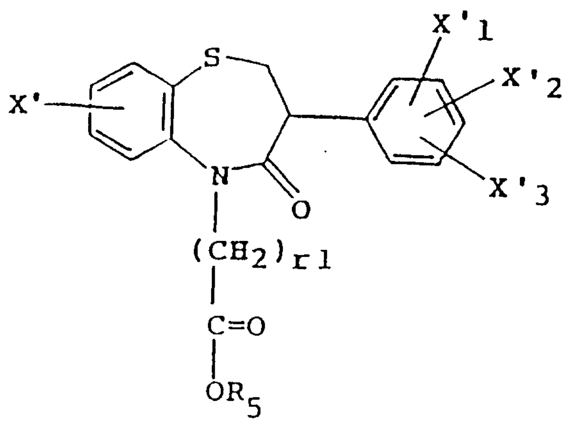

(XiX)

das man einer Hydrolyse unterwirft, wodurch man die Verbindung mit der Formel (XX) erhält:

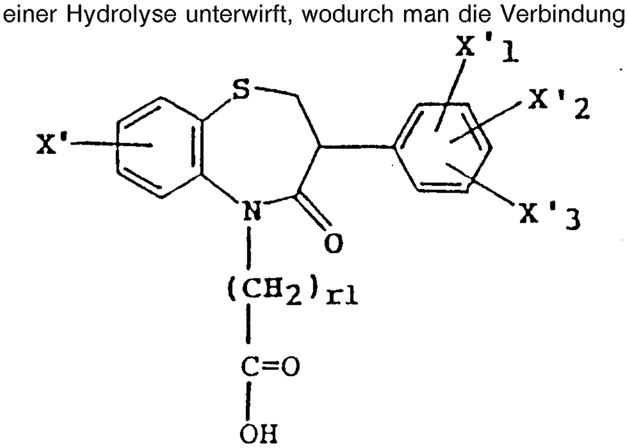

(XX)

die man mit einem Amin mit der Formel (IX) reagieren läßt, wie es oben definiert ist, wodurch man

ein Produkt der Formel (XVII) erhält, wie sie oben definiert ist, daß man dann, wenn es notwendig oder erwünscht ist, die Produkte mit der Formel (X), (XI) und (XVII), die man in racemischer oder optisch aktiver Form erhalten hat, einer oder mehrerer der folgenden Reaktionen in beliebiger Reihenfolge unterwirft:

a) Abtrennung der Schutzgruppe von der oder den geschützten reaktiven Gruppe(n) durch Hydrolyse oder Hydrogenolyse,

b) Auftrennung der racemischen Produkte mittels klassischer Verfahren, um die optisch aktiven Produkte zu erhalten,

c) Salzbildung, um die entsprechenden Salze zu erhalten.

**10.** Die Produkte mit der Formel (I), wie sie in irgendeinem der Ansprüche 1 bis 7 definiert sind, sowie ihre Additionssalze mit pharmazeutisch verwendbaren Säuren, als Arzneimittel.

**11.** Die Produkte nach Anspruch 8, sowie ihre Additionssalze mit den pharmazeutisch verwendbaren Säuren, als Arzneimittel.

**12.** Die pharmazeutischen Zusammensetzungen, die als Wirkstoff zumindest eines der Arzneimittel enthalten, wie sie in den Ansprüchen 10 und 11 definiert sind.

**13.** Die Zwischenprodukte, die den Formeln (V), (VI), (VII), (VIII), (XIX) und (XX) entsprechen, wie sie in Anspruch 9 definiert sind, als neuartige industrielle Produkte.